(19) **Europäisches Patentamt / European Patent Office / Office européen des brevets**

(11) **EP 3 659 189 B1**

(12) **FASCICULE DE BREVET EUROPEEN**

(45) Date de publication et mention de la délivrance du brevet:
**26.05.2021 Bulletin 2021/21**

(21) Numéro de dépôt: **18753092.8**

(22) Date de dépôt: **27.07.2018**

(51) Int Cl.:
*H01L 41/09* (2006.01)    *B25J 18/00* (2006.01)
*H02N 2/00* (2006.01)

(86) Numéro de dépôt international:
**PCT/EP2018/070462**

(87) Numéro de publication internationale:
**WO 2019/020804 (31.01.2019 Gazette 2019/05)**

(54) **DISPOSITIF D'ACTIONNEMENT AMPLIFICATEUR DE MOUVEMENT**

BEWEGUNGSVERSTÄRKENDE BETÄTIGUNGSVORRICHTUNG

MOVEMENT AMPLIFYING ACTUATION DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.07.2017 FR 1757122**

(43) Date de publication de la demande:
**03.06.2020 Bulletin 2020/23**

(73) Titulaires:
- **Centre National de la Recherche Scientifique
  75016 Paris (FR)**
- **Université de Franche-Comté
  25030 Besançon Cedex (FR)**
- **ENSMM - Ecole Nationale Supérieure de Mécanique et
  des Microtechniques
  25030 Besancon (FR)**

(72) Inventeurs:
- **LESCANO, Sergio
  25000 Besancon (FR)**
- **ANDREFF, Nicolas
  25480 Ecole-Valentin (FR)**
- **RAKOTONDRABE, Micky
  65430 Soues (FR)**
- **RABENOROSOA, Kanty
  25170 Courchapon (FR)**
- **TAMADAZTE, Brahim
  94000 Créteil (FR)**
- **BOUDERLIQUE, Clément
  90000 Belfort (FR)**

(74) Mandataire: **Osha Liang
2, rue de la Paix
75002 Paris (FR)**

(56) Documents cités:
FR-A1- 2 362 525     US-A1- 2010 245 966
US-A1- 2014 265 731

EP 3 659 189 B1

**Description**

DOMAINE TECHNIQUE DE L'INVENTION

L'invention porte sur les dispositifs d'actionnement amplifiés, plus précisément des dispositifs miniaturisés, occupant un volume de l'ordre de la dizaine de millimètres cube.

L'invention peut trouver de nombreuses applications, notamment pour actionner une plateforme. Cette plateforme peut accueillir un miroir pour réfléchir un faisceau laser. Le faisceau laser pour être ainsi utilisé pour le marquage et/ou la gravure laser, pour le scan 3D, dans le domaine de la (micro)robotique (vision par laser par exemple), dans le domaine des télécommunications (par exemple pour un atténuateur optique variable ou un commutateur optique), ou pour des applications médicales (chirurgie endoscopique peu invasive, exploration optique avec ou sans biopsie ...).

L'invention a été précisément développée pour la microchirurgie par laser des cordes vocales, ou phonochirurgie, avec pour objectif de guider un faisceau laser de manière précise vers les cordes vocales. La phonochirurgie ainsi appelée est définie comme suit : « toutes les procédures chirurgicales qui maintiennent, rétablissent ou améliorent la voix humaine », ou encore « la science de manipuler les éléments vibratoires du larynx afin de restaurer la fonction vocale ». Cela implique l'excision de la masse tissulaire qui pourrait être une masse bénigne ou une lésion cancéreuse. Dans la pharmacie chirurgicale au laser, l'utilisation du scalpel est remplacée par l'utilisation de technologies laser.

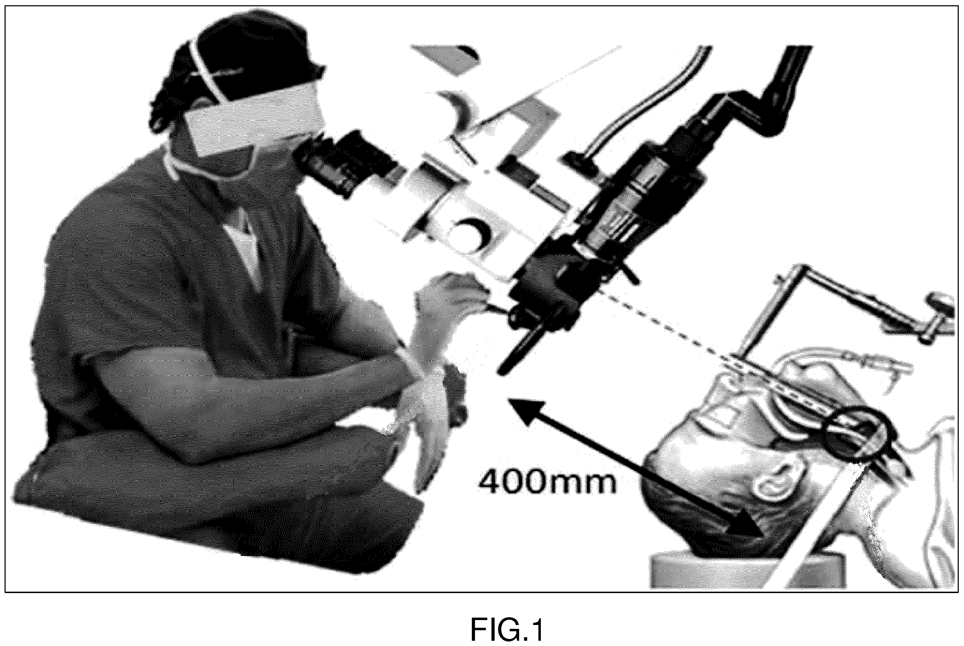

Bien qu'il existe une variété de technologies laser, la méthodologie traditionnelle répandue pour le contrôle à distance du laser chirurgical est un manipulateur mécanique, avec un microscope chirurgical positionné de manière à visualiser les cordes vocales à traiter, comme illustré en figure 1. Ce manipulateur mécanique ainsi que la source laser sont situés à environ 400 mm des cordes vocales. Cependant, plusieurs des difficultés associées à l'utilisation de ce manipulateur mécanique classique sont liées à l'ergonomie du dispositif. En conséquence, le clinicien n'a pas de moyens pratiques pour stabiliser sa main tout en effectuant les mouvements précis et délicats nécessaires pour orienter de façon précise et constante le laser chirurgical. Ainsi, il existe un risque élevé considérable de brûler des tissus sains. En outre, il existe des parties des cordes vocales qui ne sont pas accessibles.

Plus récemment, ont été développés des systèmes chirurgicaux téléopérés basé sur un microrobot en tant que système terminal d'un endoscope souple réglable (ou laryngoscope souple dans le cas des cordes vocales) pour insérer le microrobot et des caméras à l'intérieur du corps du patient, éliminant le besoin du microscope chirurgical, et permettant d'accéder aux zones non accessibles par la technique précédente. Ces nouveaux systèmes de microchirurgies laser offrent une meilleure accessibilité et une meilleure précision que les systèmes précédents. Il existe par exemple des systèmes endoscopiques mettant en œuvre la diffraction d'un laser, à l'aide de prismes ou de lentilles.

Cependant, les systèmes existants ne présentent pas un angle suffisant pour atteindre l'ensemble des cordes vocales : la direction du faisceau laser qui est gérée par réfraction (en utilisant des prismes et des lentilles) a des plages angulaires modestes (environ 2 degrés).

En outre, ils ne permettent pas de délivrer un diamètre uniforme, en effet, par réfraction, le diamètre du spot laser sur les cordes vocales change selon la direction de réfraction.

Enfin ils ne permettent pas d'avoir un contrôle précis de la position du laser : en effet, pour avoir un contrôle précis de la position du laser, il faut en général pouvoir apercevoir la position du spot laser au niveau des cordes vocales. Cependant, les lasers chirurgicaux sont tous invisibles à l'œil nu (soit ultraviolet soit infrarouge). Pour remédier à cela, on utilise un laser visible qui vient s'aligner à la direction du laser invisible. Cependant un nouveau problème apparaît : on fait passer les deux tirs lasers à travers des prismes pour les guider or les prismes prennent en compte la longueur d'onde de la lumière qui passe à travers lui pour la dévier. Ici, les deux lasers que l'on veut aligner n'ont pas la même longueur d'onde et ne sont donc pas guidés de la même façon par le prisme. On ne verra donc pas la zone exacte où le laser invisible opère.

Les inventeurs ont donc préféré opter pour un système basé sur la réflexion d'un laser à l'aide d'un miroir. Le faisceau laser est réfléchi par un miroir plan situé sur la plateforme d'un microrobot. Dans la configuration idéale, le faisceau laser sera amené par une fibre optique de l'extérieur jusqu'à l'extrémité distale du laryngoscope. Dans ce cas, un miroir est placé afin de focaliser le faisceau laser et, en même temps, de le refléter sur la plateforme du microrobot. Sur la plateforme, un miroir plan reflète ce faisceau laser sur les cordes vocales. L'orientation du miroir plan est guidée par le microrobot et elle est réalisée par une commande à distance. Ainsi, le microrobot dirige le laser sur les cordes vocales.

Ainsi, l'objectif est de développer un robot complexe apte à répondre aux nombreuses contraintes qu'une telle intervention chirurgicale soulève. En particulier, le robot devait répondre aux critères suivants :

- deux degrés de liberté au moins pour pouvoir intervenir sur l'ensemble des cordes vocales ;
- distance entre la tête du laryngoscope souple et les cordes vocales de 20 mm, impliquant une portée minimale à développer par le faisceau laser est de $\pm$ 25° pour chaque axe (environ un cercle de 20 mm de diamètre), ce qui correspond à une plage angulaire de $\pm$ 12,5° pour chaque axe de rotation du miroir ;
- résolution du balayage laser sur la corde vocale

meilleure que 100 $\mu$m, et ce pour ne pas risquer d'abimer des cellules saines en traitant des cellules malignes : cela équivaut à environ 0,15° de résolution dans chaque angle de rotation du miroir à la distance de référence de 20 mm ;

- bande passante d'environ 200Hz au moins : la vitesse de déplacement du faisceau et donc du miroir soit être suffisante pour ne pas risquer d'endommager les tissus sains des cordes vocales ; en effet, le laser étant un système d'ablation de cellules à traiter et ayant une puissance élevée, il ne doit pas rester longtemps au même endroit pour ne pas abîmer les cellules voisines qui pourraient être des cellules saines ; un temps de passage maximum doit donc être respecté exprimé sous la forme d'une bande passante minimum ;

- biocompatibilité : comme le laryngoscope flexible et la tête qui renferme le microrobot visent à travailler dans des espaces in vivo, l'appareil doit utiliser des matériaux soit biocompatibles, soit protégés par des matériaux biocompatibles pour s'assurer qu'il ne soit pas en contact avec les tissus humains ;

- système de mise au point : pour permettre une bonne détection du spot laser par les caméras et permettre une bonne résolution sur les cordes vocales, son diamètre spécifié est de 200 $\mu$m ;

- taille du microrobot : il doit être inclus dans un cube inférieur à 10 x 10 x 10 mm$^3$.

Ce robot complexe doit remplir a minima les deux fonctions que sont : la fonction d'orientation du miroir selon deux axes de rotation et dans la plage angulaire recherchée, et la fonction d'actionnement à distance desdites rotations pour atteindre la plage angulaire recherchée, avec une précision (résolution) et une vitesse minimale de déplacement (bande passante) respectant les critères sus mentionnés.

L'invention concerne la seconde fonction, c'est-à-dire à la fonction d'actionnement à distance desdites rotations.

Ainsi l'objectif est de disposer d'un dispositif d'actionnement qui permette d'actionner un objet selon une amplitude minimum donnée, en précision et en vitesse, ce dispositif d'actionnement devant être inclus dans un cube inférieur à 10 x 10 x 10 mm$^3$.

ETAT DE LA TECHNIQUE

Les dispositifs d'actionnement (ou actionneurs) transforment un type d'énergie électrique, thermique, magnétique ou autre en mouvements ou en charges mécaniques. Les actionneurs sont des composants clés de la robotique. Habituellement, dans les micro-mécanismes, le mot utilisé pour désigner un actionneur est le «micro-actionneur». Les micro-actionneurs sont souvent constitués de matériaux actifs ou intelligents qui doivent fournir typiquement une résolution micrométrique ou submicrométrique. Les matériaux actifs ont des caractéristiques recherchées telles que la haute résolution et/ou la bande passante élevée. En outre, la plupart d'entre eux peuvent être utilisés également comme détecteurs ou capteurs.

Les dimensions d'un micro-actionneur sont généralement inférieures à 10 mm.

Les micro-actionneurs les plus adaptés aux critères sus-mentionnés sont des micro-actionneurs piézoélectriques. On utilise le phénomène de piézoélectricité qui fait apparaître des charges électriques sur les surfaces d'un matériau lorsqu'il est exposé à une charge mécanique - c'est ce qu'on appelle l'effet piézoélectrique direct - et inversement une déformation est obtenue lorsqu'un champ électrique est appliqué. C'est cet effet inverse qui est exploité pour un actionneur.

Les micro-actionneurs piézoélectriques offrent une haute résolution (jusqu'à quelques nanomètres), une bande passante élevée (jusqu'à des dizaines ou centaines de kilohertz) et une densité de force élevée par rapport à d'autres matériaux intelligents, ce qui les rend adaptés pour la conception de micro-actionneurs.

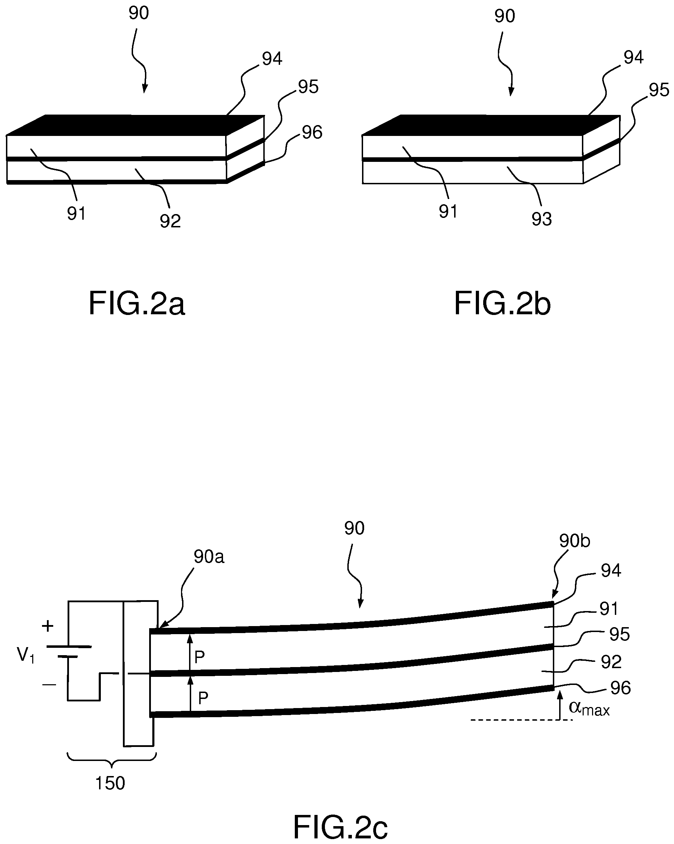

Comme illustré en figures 2a, 2b et 2c, ce sont des structures 90 de section rectangulaire, préférentiellement configurées en porte-à-faux.

Ces structures comprennent différentes couches ou lames collées l'une contre l'autre, on parle aussi de structure bilame lorsqu'il y a deux lames.

Ces structures comprennent une couche active 91, comprenant un matériau piézoélectrique, la couche active 91 étant prise entre deux électrodes 94, 95 aptes à exciter le matériau piézoélectrique actif. Dans ce cas, on parle de structure unimorphe (figure 2b).

Une structure unimorphe peuvent comprendre une couche supplémentaire, dite couche passive, qui peut être une couche d'un matériau souple ou flexible, et qui peut être fixée contre une des électrodes. Dans une structure unimorphe, une couche passive permet de générer la courbure exploitable de la poutre lors de l'alimentation de la couche active. Cette couche passive peut être conductrice et fonctionner comme électrode.

Ces structures peuvent comprendre plusieurs couches actives, par exemple. On parle de structure bimorphe s'il y a au moins deux couches actives 91, 92 (figure 2a). Chaque couche active 91, 92 est prise entre deux électrodes 94, 95, 96 aptes à exciter le matériau piézoélectrique actif. Les poutres bimorphes permettent de plus grands déplacements. On parle plus généralement de structure multimorphe s'il y a deux ou plus de couches actives.

Une structure multimorphe peut également comprendre une couche supplémentaire, dite couche passive. La couche passive dans une structure multimorphe aide à augmenter la rigidité de la structure.

Des moyens 150 doivent être prévus pour alimenter électriquement les électrodes et exciter les matériaux piézoélectriques.

Une telle structure 90 peut être nommée : poutre piézoélectrique, ou poutre comprenant un élément piézoélectrique.

Pour être configurée en porte-à-faux, comme illustré en figure 2c, l'une des extrémités 90a de la poutre 90 est fixée, et l'autre 90a décrit un mouvement de flexion de part et d'autre d'une position de référence dite position de repos. Une poutre piézoélectrique peut ainsi fléchir autour de ladite position de repos, jusqu'à un angle maximum $\alpha_{max}$ correspondant à une amplitude de flexion d'environ 150 à 200 $\mu$m pour une poutre de 10mm x 2mm x 0,3mm d'un matériau piézoélectrique PZT-5A, et en structure bimorphe.

L'amplitude du mouvement de flexion (ou l'angle $\alpha_{max}$) est fonction de

- la dimension de la poutre (de la longueur et de son épaisseur ; la largeur permettant plutôt de dimensionner la force applicable) ;
- la morphologie de la poutre (unimorphe, bimorphe ...)
- la tension appliquée entre les électrodes.

Plus l'amplitude du mouvement de flexion (ou l'angle $\alpha_{max}$) recherchée sera grande, plus la vitesse (ou la fréquence) de mouvement faible et inversement. En d'autres termes, les micro-actionneurs connus ne répondent pas à la double contrainte d'amplitude et de vitesse (ou de fréquence).

Or il est recherché des amplitudes d'ordre millimétrique, tout en minimisant les dimensions de la poutre et la tension appliquée, ceci en gardant une bonne dynamique de mouvement de la poutre.

Il a donc été recherché des amplificateurs de mouvement.

Le document de brevet FR2850218 décrit un actionneur piézoélectrique à déplacement amplifié comprenant un amplificateur mécanique de déplacement relié à une charge et à une base, et présentant une forme de coquille en ellipse réalisée dans un matériau élastique déformable, et des éléments piézoélectriques montés à l'intérieur de ladite coquille dans la direction du grand axe de la coquille, et excités électriquement pour produire une déformation longitudinale dudit grand axe et induire une déformation du petit axe destinée à générer à l'interface avec la charge un déplacement dont la composante le long du petit axe est amplifié Le document FR2362525 décrit un moteur piézo-électrique pas-à-pas, composé d'un élément de guidage fixe, d'une colonne piézo-électrique mobile sur celui-ci et servant d'élément d'entraînement, et d'un dispositif piézo-électrique de serrage disposé sur la colonne.

Le document de brevet précité met en œuvre des actionneurs piézoélectriques de type stack, c'est-à-dire utilisant une autre forme d'actionnement piézoélectrique. Les actionneurs piézoélectriques stacks n'utilisent pas l'effet bilame, mais un déplacement directement obtenu par la déformation du matériau piézoélectrique. Les dimensions des stacks sont relativement grandes, et les déplacements générés sont très faibles, toutefois ils ont une force beaucoup plus grande que les poutres bilames. Ils ont pour but d'améliorer les capacités d'amortissement d'un actionneur piézoélectrique et sa tenue aux efforts extérieurs dynamiques.

En outre l'actionneur piézoélectrique à déplacement amplifié selon ce document de brevet est un système contraint en termes d'amplitude et de vitesse de déplacement de par sa structure en coquille.

Il existe donc un réel besoin de disposer d'un dispositif d'actionnement, notamment d'un micro-actionneur présentant à la fois une grande vitesse de déplacement (par exemple une vitesse correspondant à un temps de réponse de 1ms) et une amplitude de flexion importante (par exemple de l'ordre du mm), c'est-à-dire beaucoup plus que ne le permettent les micro-actionneurs connus.

En outre, les amplitudes délivrées doivent également être précises et doivent pouvoir être maîtrisées avec une précision par exemple de l'ordre de 50 $\mu$m pour 1 mm.

EXPOSE DE L'INVENTION

Pour résoudre le problème précité, l'invention a pour objet un dispositif d'actionnement amplificateur de mouvement caractérisé en ce qu'il comprend :

- une première poutre comprenant un élément piézoélectrique, apte à fléchir selon un axe principal lorsqu'une tension lui est appliquée et apte à être attachée en une première extrémité à un premier point fixe ;
- une seconde poutre comprenant un élément piézoélectrique, apte à fléchir selon ledit axe principal lorsqu'une tension lui est appliquée, et présentant une première extrémité et une seconde extrémité ;
- une première articulation comprenant

  ▪ une première partie flexible selon un premier axe perpendiculaire à l'axe principal et reliée à la première poutre en une seconde extrémité de ladite première poutre,
  ▪ une deuxième partie flexible selon un second axe perpendiculaire à l'axe principal et reliée à la seconde poutre en la première extrémité de ladite seconde poutre,
  ▪ une première partie rigide reliant les première et deuxième parties flexibles,
  ▪ une seconde partie rigide apte à être positionnée contre un deuxième point fixe,
  ▪ une troisième partie flexible selon un troisième axe perpendiculaire à l'axe principal reliant la seconde poutre à la seconde partie rigide en un point de pivot de ladite seconde poutre, de sorte que l'ensemble formé par la seconde partie rigide et la seconde poutre forme un levier autour dudit point de pivot,

lesdites parties flexibles et rigides étant les parties d'une pièce d'un seul tenant.

Par « articulation », il faut comprendre un moyen d'assemblage de deux poutres qui permette une articulation selon un ou deux degrés de liberté. Cette articulation permet que les mouvements des poutres soient amplifiés.

Le dispositif est configuré de telle sorte que les effets cumulés de la flexion reçue par le segment compris entre le point de pivot et la première extrémité de la seconde poutre et de la flexion de ladite seconde poutre lorsqu'une tension lui est appliquée génère un mouvement de flexion amplifié au segment compris entre le point de pivot et la première extrémité de ladite seconde poutre.

La première extrémité de ladite seconde poutre est une extrémité libre, qui peut être reliée à un système à actionner.

Dans certains modes de réalisation, le dispositif d'actionnement amplificateur de mouvement comprend en outre :

- une troisième poutre comprenant un élément piézo-électrique, apte à fléchir selon l'axe principal lorsqu'une tension lui est appliquée, et présentant une première extrémité et une seconde extrémité ;
- une seconde articulation comprenant :

    ■ une première partie flexible selon un axe perpendiculaire à l'axe principal et reliée à la seconde poutre en la seconde extrémité de ladite seconde poutre,
    ■ une deuxième partie flexible selon un axe perpendiculaire à l'axe principal et reliée à la troisième poutre en la première extrémité de ladite troisième poutre,
    ■ une première partie rigide reliant les première et deuxième parties flexibles,
    ■ une seconde partie rigide apte à être positionnée contre un troisième point fixe,
    ■ une troisième partie flexible selon un troisième axe perpendiculaire à l'axe principal reliant la troisième poutre à la seconde partie rigide en un point de pivot de ladite troisième poutre, de sorte que l'ensemble formé par la seconde partie rigide et la seconde poutre forme un levier autour dudit point de pivot,
    lesdites parties flexibles et rigides étant les parties d'une pièce d'un seul tenant.

Dans cette configuration, trois poutres sont reliées entre elles par deux articulations à une ou deux degrés de liberté.

Dans cette configuration, la troisième poutre présente une première extrémité libre, qui peut reliée à un système à actionner.

Cette configuration est particulièrement intéressante en ce qu'elle permet d'amplifier la flexion reçue par la troisième poutre, la deuxième poutre formant amplificateur intermédiaire.

Dans certains modes de réalisation, le dispositif d'actionnement amplificateur de mouvement comprend :

- une première poutre comprenant un élément piézoélectrique, et étant apte à fléchir selon un axe principal lorsqu'une tension lui est appliquée, et apte à être attachée en une première extrémité à un premier point fixe;
- N autres poutres, N étant supérieur ou égal à 2 et M variant entre 2 et N, chaque M-ième poutre comprenant un élément piézo-électrique, et étant apte à fléchir selon l'axe principal lorsqu'une tension lui est appliquée, et présentant une première extrémité et une seconde extrémité ; la N-ième poutre présentant une seconde extrémité libre ;
- X articulations, X étant égal à N-1 et Y étant égal à M-1, chaque Y-ième articulation comprenant :

    ■ une première partie flexible selon un axe perpendiculaire à l'axe principal et reliée à la Y-ième poutre en la seconde extrémité de ladite Y-ième poutre,
    ■ une deuxième partie flexible selon un axe perpendiculaire à l'axe principal et reliée à la M-ième poutre en la première extrémité de ladite M-ième poutre,
    ■ une première partie rigide reliant les première et deuxième parties flexibles,
    ■ une seconde partie rigide apte à être positionnée contre un M-ième point fixe,
    ■ une troisième partie flexible selon un axe perpendiculaire à l'axe principal reliant la M-ième poutre à la seconde partie rigide en un point de pivot de ladite M-ième poutre, de sorte que l'ensemble formé par la seconde partie rigide et la M-ième poutre forme un levier autour dudit point de pivot.

Dans cette configuration, plusieurs poutres sont reliées entre elles par une triple articulation à une ou deux degrés de liberté.

Dans cette configuration, la première poutre est reliée en une première extrémité à un point fixe et la N-ième poutre présente une première extrémité libre.

Ladite extrémité libre de la N-ième poutre peut être reliée à un système à actionner.

Cette configuration est encore plus particulièrement intéressante en ce qu'elle permet d'amplifier la flexion reçue par la dernière poutre.

En outre, il peut être envisagé de réaliser des câblages communs, même si le système perd un degré de liberté en actionnement.

Il peut également être envisagé de ne pas actionner certaines poutres qui conservent une capacité mécanique d'amplification. En actionnant ou pas certaines poutres de la structure active, il pourrait être possible de gérer et/ou d'affiner la précision, le temps de réponse et/ou l'amplitude de flexion.

Dans certains modes de réalisation, une Y-ième articulation comprend en outre une troisième et une quatrième parties rigides formant avec les autres parties de ladite articulation une pièce d'un seul tenant :

- la troisième partie rigide formant la liaison entre la première partie flexible de ladite Y-ième articulation et la Y-ième poutre,
- la quatrième partie rigide formant la liaison entre la deuxième partie flexible de ladite Y-ième articulation avec la M-ième poutre.

Dans certains modes de réalisation, les parties flexibles d'au moins une Y-ième articulation présentent des axes d'articulation parallèles entre eux et perpendiculaires à l'axe principal.

Dans certains modes de réalisation, l'élément piézoélectrique comprend du titanate de zirconate au plomb. Le titanate de zirconate au plomb (PZT) est un matériau piézoélectrique, qui est avantageux car sa biocompatibilité a été prouvée sous certaines conditions.

Dans certains modes de réalisation, une poutre présente une structure bimorphe. Elle comprend dans ce cas deux éléments piézoélectriques. Cela permet d'avoir de plus amples flexions sur une poutre.

L'invention concerne également un microrobot à cinématique parallèle sphérique à deux degrés de liberté comprenant :

- un dispositif d'orientation sphérique à deux degrés de liberté comprenant une plateforme à orienter selon un premier et un second axes de rotation par rapport à une base fixe, et un premier et un second bras d'actionnement ;
- un premier et un deuxième dispositifs d'actionnement amplificateur de mouvement selon l'invention ;

  ▪ le premier dispositif d'actionnement étant connecté au premier bras d'actionnement de manière à lui transmettre un premier mouvement de translation relativement à la base fixe de sorte à entraîner la plateforme en rotation selon le premier axe de rotation,

  ▪ et le deuxième dispositif d'actionnement étant connecté au second bras d'actionnement de manière à lui transmettre un second mouvement de translation relativement à la base fixe de sorte à entraîner la plateforme en rotation selon le second axe de rotation.

Dans certains modes de réalisation, le dispositif d'orientation est un dispositif d'orientation sphérique à deux degrés de liberté reliant une plateforme à deux points de fixation d'une base fixe de manière à pouvoir orienter ladite plateforme dans l'espace par rotation autour d'un premier axe et d'un second axe, ces deux axes étant sensiblement perpendiculaires et se croisant en un centre de mouvement sphérique situé dans ledit organe à orienter, comprenant :

- un premier bras d'actionnement configuré pour effectuer un mouvement de translation relativement à la base fixe et apte à appliquer à un premier bras de transmission, relié à un premier point de fixation de la base fixe par une liaison flexible articulée selon le premier axe, un mouvement de rotation relativement à ladite base fixe de manière à transmettre à la plateforme un mouvement de rotation selon ledit premier axe ;
- un bras intermédiaire relié au premier bras de transmission par une liaison flexible articulée autour d'un troisième axe, perpendiculaire aux premier et second axes, et relié à la plateforme par une liaison flexible, de manière à transmettre à la plateforme un mouvement de rotation selon le premier axe ;
- un second bras d'actionnement configuré pour effectuer un mouvement de translation relativement à la base fixe et apte à appliquer à un second bras de transmission, relié à un second point de fixation de la base fixe par une liaison flexible articulée selon le second axe, un mouvement de rotation relativement à ladite base fixe de manière à appliquer sur la plateforme un mouvement de rotation selon ledit second axe, ledit bras étant relié à la plateforme par une liaison flexible articulée selon le premier axe, de manière à ne pas entraîner ledit second bras de transmission en rotation selon le premier axe lors de l'actionnement du premier bras d'actionnement ;

la liaison entre la plateforme et le bras intermédiaire étant articulée selon l'axe de manière à ne pas entraîner ledit bras intermédiaire en rotation selon le second axe lors de l'actionnement du second bras d'actionnement ; et les bras, les liaisons flexibles et la plateforme formant les parties d'une pièce formée en un seul tenant formant le dispositif, les bras et la plateforme étant des parties rigides du dispositif, et les liaisons flexibles étant des parties flexibles formant chacune une charnière selon un seul des premier, second et troisième axes, et reliant lesdites parties rigides entre elles, à la base fixe.

Dans certains modes de réalisation, les parties rigides comprennent une couche centrale d'un matériau flexible, tel un polyimide, prise en sandwich entre deux couches d'un matériau rigide, telle de la fibre de carbone, les liaisons flexibles étant composées de la couche centrale.

Dans certains modes de réalisation, un ou des bras de transmission et/ou bras intermédiaire forment un arc de cercle.

Dans certains modes de réalisation, le dispositif d'orientation sphérique comprend en outre une liaison flexible articulée autour d'un axe parallèle au premier axe et disposée entre le premier bras d'actionnement et le premier bras de transmission, et formant avec les bras, les liaisons flexibles et la plateforme une pièce d'un seul tenant.

Dans certains modes de réalisation, le premier bras d'actionnement comprend une première partie apte à être couplée au dispositif d'actionnement selon l'invention et une seconde partie reliée au premier bras de transmission par une liaison flexible articulée autour d'un axe parallèle au premier axe, les lesdites parties première et seconde parties étant reliées par une liaison flexible articulée selon un axe parallèle au premier axe et formant avec les bras, les liaisons flexibles et la plateforme une pièce d'un seul tenant.

Dans certains modes de réalisation, le dispositif d'orientation sphérique comprend en outre une liaison flexible reliant le second bras d'actionnement et le second bras de transmission, articulée autour d'un axe parallèle au second axe et formant avec les bras, les liaisons flexibles et la plateforme une pièce d'un seul tenant.

Dans certains modes de réalisation, le dispositif d'orientation sphérique comprend en outre une double liaison flexible de type cardan reliant le second bras d'actionnement et le second bras de transmission, articulée autour d'un axe parallèle au premier axe et d'un axe parallèle au second axe, et formant avec les bras, les liaisons flexibles et la plateforme une pièce d'un seul tenant.

Dans certains modes de réalisation, le second bras d'actionnement comprend une première partie apte à être couplée à un dispositif d'actionnement selon l'invention et une seconde partie reliée au second bras de transmission par une simple liaison flexible simple ou une double liaison flexible, les première et seconde parties étant reliées une double liaison flexible de type cardan articulée autour d'un axe parallèle au premier axe et d'un axe parallèle au second axe, et formant avec les bras, les liaisons flexibles et la plateforme une pièce d'un seul tenant.

Dans certains modes de réalisation, la plateforme du dispositif d'orientation sphérique supporte un miroir.

Dans certains modes de réalisation, le miroir est disposé de manière à réfléchir un faisceau laser.

Dans certains modes de réalisation, le microrobot comprend en outre un dispositif de visualisation d'un spot du faisceau laser positionné sur une surface.

DESCRIPTION DES FIGURES

L'invention sera mieux comprise et d'autres avantages apparaîtront à la lecture de la description suivante qui est donnée à titre illustratif et non limitatif, et à la vue des figures suivantes annexées :

- la figure 1 illustre une méthode de microchirurgie laser selon l'état de la technique
- les figures 2a, 2b et 2c illustrent des structures piézoélectriques bimorphe ou unimorphe connues de l'état de la technique ;
- les figures 3a et 3b schématisent deux exemples de dispositif d'actionnement selon l'invention en vue 3D, le premier avec deux poutres et le second avec trois poutres ;
- les figures 4a et 4b schématisent les deux mêmes exemples de dispositif d'actionnement, avec leurs mouvements de flexion amplifiés en vue 2D, le premier avec deux poutres et le second avec trois poutres ;
- la figure 5 illustre le détail d'une partie flexible entre deux parties rigides d'un dispositif d'actionnement selon l'invention ;
- les figures 6a et 6b illustrent le dimensionnement d'une partie flexible entre deux parties rigides illustré en figure 5 ;
- les figures 7a, 7b, 7c, 7d et 7e illustrent un exemple de procédé de fabrication du détail illustré en figure 5 ;
- les figures 8a, 8b et 8c présentent des articulations selon l'invention ;
- les figures 9a, 9b illustrent un exemple d'un dispositif d'actionnement selon l'invention, en vue 3D selon deux angles différents ;
- la figure 10 illustre un exemple de dispositif d'actionnement selon l'invention, en vue 2D ;
- les figures 11a, 11b illustrent un exemple de microrobot selon l'invention, sans support et selon deux vues différentes en 3D ;*
- les figures 11c, 11d illustrent un exemple de microrobot selon l'invention, avec support et selon deux vues différentes en 3D ;
- la figure 12 illustre un premier exemple d'application du microrobot selon l'invention ;
- la figure 13 illustre un second exemple d'application du microrobot selon l'invention.

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.

EXPOSÉ DÉTAILLÉ DE MODES DE RÉALISATION PARTICULIERS

La figure 1 illustre une méthode de microchirurgie laser selon l'état de la technique qui a été décrite en introduction de la présente demande et ne sera pas reprise ici.

Les figures 2a, 2b et 2c qui illustrent des exemples de poutres piézoélectriques bimorphe ou unimorphe connues de l'état de la technique ont été décrites en introduction de la présente demande et ne seront pas reprises ici.

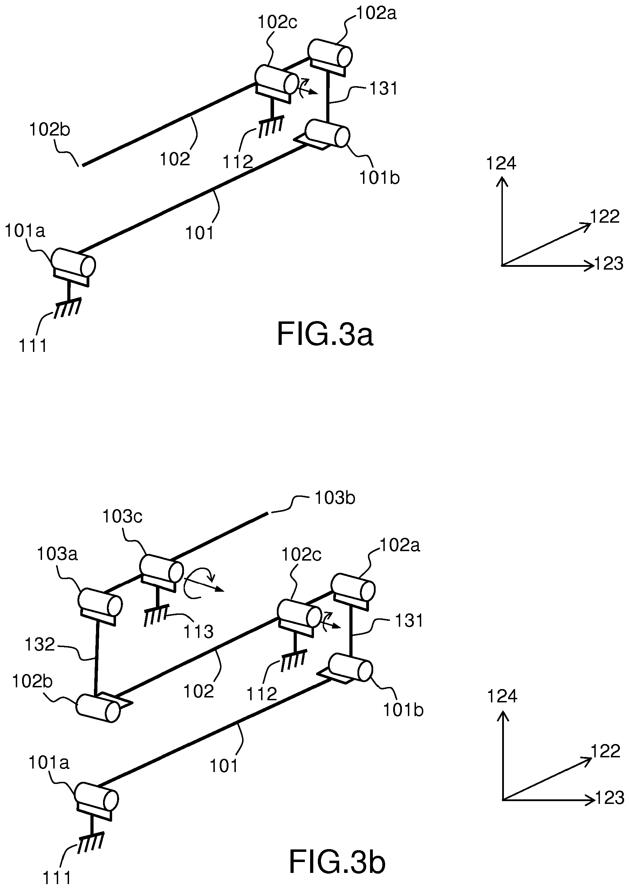

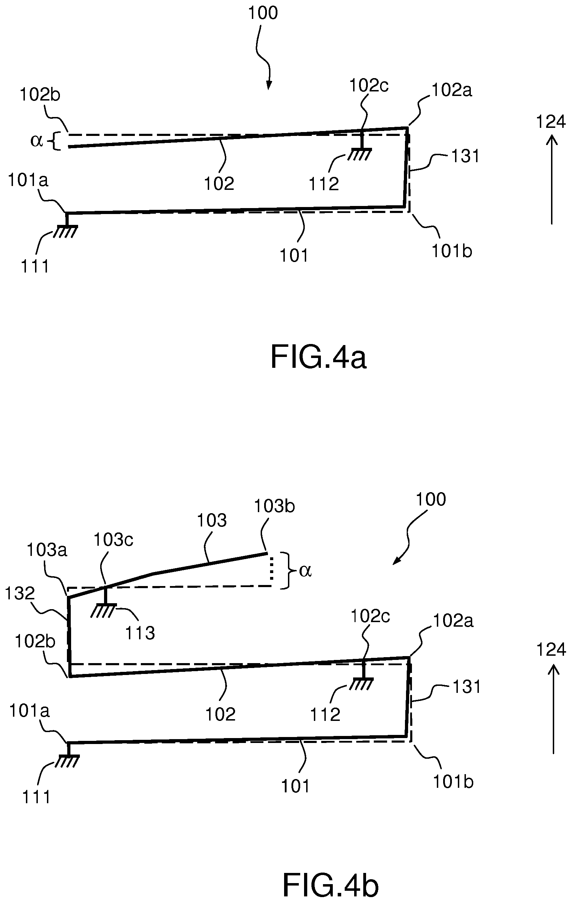

Les figures 3a et 3b fournissent des schémas de principe de deux dispositifs d'actionnement selon l'invention en vue 3D, le premier avec deux poutres et le second avec trois poutres. Elles seront décrites en même temps que les figures 4a et 4b qui schématisent les deux mêmes exemples, et indiquent en outre les mouvements de flexion en vue 2D. Ces figures sont des vues cinématiques.

Le dispositif d'actionnement représenté en figures 3a et 4a comprend une première poutre piézoélectrique 101 apte à fléchir selon un axe principal 124 lorsqu'une tension lui est appliquée et attachée en une première extrémité 101a à un point fixe 111, et une seconde poutre piézoélectrique 102 également apte à fléchir selon l'axe principal 124 lorsqu'une tension lui est appliquée. Une seconde extrémité 102b de la seconde poutre 102 est laissée libre, une première extrémité 102a de la seconde poutre 102 est reliée à la première poutre 101 par l'intermédiaire d'une première articulation 131.

Ainsi, le dispositif d'actionnement 100 comprend une première articulation 131 à un ou deux degrés de liberté relie les première et deuxième poutres 101 et 102. Elle permet également de positionner la deuxième poutre 102 contre un point fixe 112, ce qui permet d'obtenir un effet de levier au niveau d'un point 102c de la deuxième poutre 102.

Dans l'exemple représenté, la première articulation 131 ne comprend qu'un degré de liberté. Autrement dit, elle ne permet une rotation entre les poutres 101 et 102 que selon un axe, dans cet exemple l'axe 122 perpendiculaire à l'axe principal 124.

Le dispositif d'actionnement représenté en figures 3b et 4b comprend en outre une troisième poutre piézoélectrique 103 apte à fléchir selon l'axe principal 124 lorsqu'une tension lui est appliquée. Une seconde extrémité 103b de la troisième poutre 103 est laissée libre. Dans ce cas, la seconde extrémité 102b de la seconde poutre 102 n'est plus laissée libre et elle est reliée à la troisième poutre 103 par l'intermédiaire d'une seconde articulation 132.

Ainsi, le dispositif d'actionnement 100 comprend une seconde articulation 132 à un ou deux degrés de liberté relie les deuxième et troisième poutres 102 et 103. Elle permet également de positionner la troisième poutre 103 contre un point fixe 113, ce qui permet d'obtenir un effet de levier au niveau d'un point 103c de la troisième poutre 103.

Dans l'exemple représenté, la seconde articulation 132 ne comprend qu'un degré de liberté. Autrement dit, elle ne permet une rotation entre les poutres 102 et 103 que selon un axe, dans cet exemple l'axe 122 perpendiculaire à l'axe principal 124.

Cette configuration est particulièrement intéressante en ce qu'elle permet d'amplifier la flexion reçue par la troisième poutre 103, la deuxième poutre 102 formant amplificateur intermédiaire.

Les articulations 131 et 132 sont réalisées grâce à des liaisons particulières, dites liaisons flexibles, qui seront décrites plus loin.

Le dispositif d'actionnement 100 selon l'invention est constitué d'un ensemble d'articulations 131 (respectivement 132) à un ou deux degrés de libertés reliant deux poutres piézoélectriques 101 et 102 (respectivement 102 et 103), chacune de ces articulations étant une pièce d'un seul tenant, et étant formée de liaisons flexibles simples et de parties rigides chaque partie rigide étant positionnée entre deux parties flexibles.

Il est donc important de comprendre comment est configurée et fabriquée une liaison flexible simple, qui constitue la base de la fabrication du dispositif d'actionnement 100 selon l'invention.

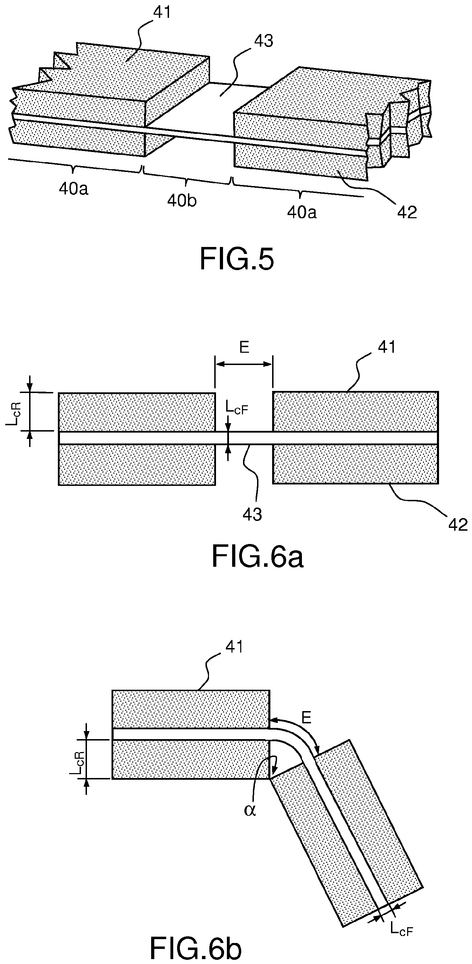

La figure 5 illustre le détail d'une liaison flexible, qui représente un détail d'un dispositif d'actionnement selon l'invention.

Une liaison flexible 40, qui peut également être nommée joint pliable, se définit comme une charnière de flexion formée par une partie plus mince 40b d'une pièce, de sorte à fournir une rotation relative entre deux parties rigides 40a de la même pièce, adjacentes à ladite partie plus mince 40b.

Dans l'exemple illustré, la pièce comprend une couche flexible 43 en polyimide et une première et deuxième couches rigides 41, 42 en fibres de carbone. La partie flexible 40b correspond à largeur de la couche flexible 43 en polyimide qui n'est pas prise entre les deux première et deuxième couches rigides 41, 42. Cela correspond à un évidement qui a été pratiqué sur les couches rigides 41 et 42, comme cela est expliqué plus loin.

Les figures 6a et 6b illustrent le dimensionnement d'une liaison flexible 40. Une liaison flexible, ou joint pliable, peut être dimensionnée, en jouant sur l'écart E qui correspond à la largeur de la couche flexible 43 qui n'est pas prise entre les deux couches rigides 41 et 42 et sur la somme de l'épaisseur $L_{CR}$ d'une couche rigide 41 et de l'épaisseur $L_{CF}$ de la couche flexible 43 L'angle $\alpha_{max}$ pouvant être développé par le joint pliable 43 dépend de E et de $L_{CR}$ selon la formule suivante :

$$E = \alpha_{max} \times (L_{CR} + L_{CF}) / 2 \times f$$

où :

- $L_{CR}$ est l'épaisseur d'une couche de matériau rigide
- $L_{CF}$ l'épaisseur de la couche flexible
- f est le facteur de risque pris en compte pour éviter que la charnière ne se bloque en cas de défaut de fabrication. Il est généralement fixé à 1,1.

L'épaisseur $L_{CF}$ de la couche flexible n'est généralement pas été prise en compte car elle est souvent négligeable devant l'épaisseur $L_{CR}$ de la couche rigide.

Ainsi, ces liaisons flexibles peuvent être aisément dimensionnées pour obtenir l'angle $\alpha_{max}$ à développer.

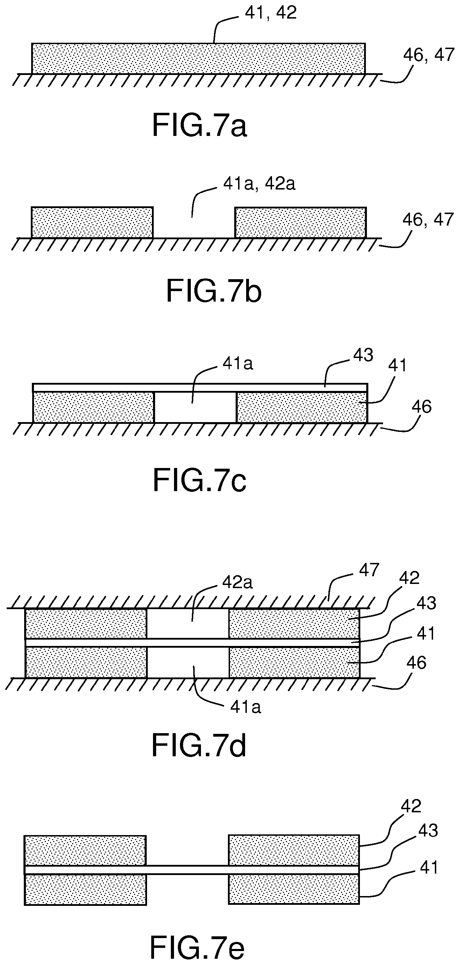

Les figures 7a à 7e montrent un exemple de procédé de fabrication d'une liaison flexible 40 illustrée en figure 5. Le procédé de fabrication comprend les étapes suivantes :

a) positionnement d'une première couche rigide 41, par exemple une couche de fibre de carbone d'une épaisseur de 130 $\mu$m, sur un premier support 46, et d'une deuxième couche rigide 42, par exemple une couche de fibre de carbone d'une épaisseur de 130 $\mu$m, sur un second support 47 (figure 7a) ;

b) micro-usinage de chaque couche rigide 41, 42 afin de créer un évidement 41a, 42a sur toute l'épaisseur de la couche rigide de carbone $L_{CR}$ et sur une largeur donnée E identique pour les deux couches rigides 41, 42 (figure 7b) ;

c) fixation, par exemple par collage, d'une couche flexible 43, par exemple une couche de polyimide de 10 $\mu$m d'épaisseur, sur la première couche rigide 41 évidée (figure 7c) ;

d) ajustement de la seconde couche rigide 42 évidée sur la couche flexible 43 fixée sur la première couche rigide 41 de manière à ce que les évidements 41a et 42a des deux couches rigides 41 et 42 se trouvent en vis-à-vis puis fixation, par exemple par collage, de la seconde couche rigide 42 sur la couche flexible 43 (figure 7d) ;

e) micro-usinage de la structure composite 40 ainsi obtenue de manière à la libérer des supports 46 et 47 (figure 7e).

La structure composite ou liaison flexible 40 ainsi obtenue peut ainsi s'articuler selon un axe de rotation comme le montre la figure 6. Ladite technique de fabrication des liaisons flexibles 40 permet d'obtenir des angles de flexion importants selon un degré de liberté.

Chaque couche rigide 41, 42 de fibre de carbone peut être obtenue par durcissement d'une feuille de fibre de carbone à l'aide d'une résine thermodurcissable chauffée dans un four.

Entre les étapes d) et e), la structure composite obtenue peut être placée dans un four et chauffée de manière à être solidifiée, et pendant cette étape une pression peut être appliquée à la structure pour maintenir la nouvelle structure unie et pour éviter les ondulations.

On utilise avantageusement le micro-usinage laser par sa capacité à fabriquer avec précision une grande variété de matériaux : la plupart des métaux, des céramiques, des plastiques, des fibres de carbone. On peut utiliser par exemple un laser femto seconde (de quelques centaines de femto secondes), ou un laser DPSS « Diode-pumped solid-state ».

On peut utiliser l'une des deux méthodes de micro-usinage au laser décrites ci-dessous :

- en focalisant le faisceau laser : le rayon laser est focalisé sur un point sur le matériau de la pièce. Ensuite, le point laser est déplacé dans les axes x, y ou z pour permettre de vaporiser des portions de celui-ci. Cette méthode est préférée car plus directe et rapide.

- par projection sur un masque : un motif de masque est placé entre la source laser et la pièce à usiner. Cette méthode permet de vaporiser uniquement les pièces non cachées. En général, il est utilisé pour des modèles superficiels. Pour obtenir une profondeur considérable, l'opération est répétée plusieurs fois.

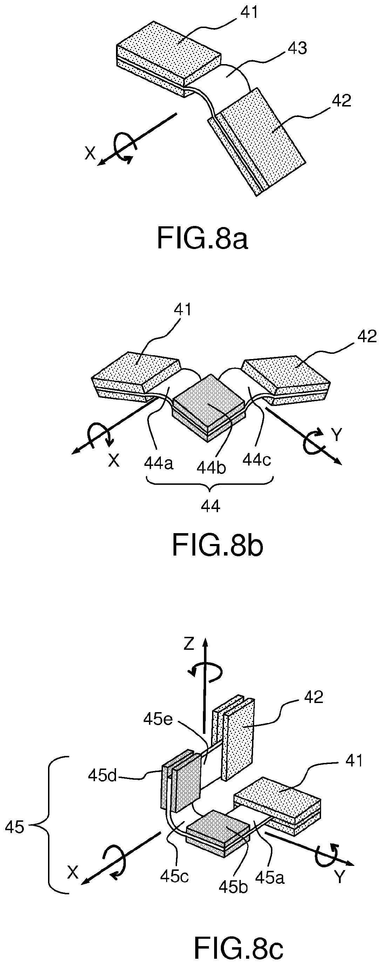

Les figures 8a, 8b et 8c présentent des articulations selon l'invention. Les articulations selon l'invention sont appelées liaisons flexibles ou joints pliables.

La fabrication du dispositif d'actionnement selon l'invention 100 selon l'invention est basée sur l'utilisation de liaisons flexibles, qui remplacent les articulations conventionnelles, comme représenté sur les figures 8a à 8c.

Ces liaisons flexibles simples peuvent être combinées et arrangées pour réaliser des articulations plus complexes.

Comme illustré en figure 8a, une liaison pivot (revolute joint) à un degré de liberté (rotation selon l'axe x) est assurée par une simple articulation ou simple liaison flexible 40 décrite précédemment en lien avec les figures 5, 6a et 6b. Ce type de liaison est nommé indifféremment « simple liaison flexible » ou « liaison flexible » dans la présente demande de brevet.

Comme illustré en figure 8b, une liaison rotule à doigt ou de type cardan (universal joint) à deux degrés de liberté (rotations selon les axes x et y) est assurée par un système combinant une première liaison flexible 44a assurant une articulation selon l'axe x entre la première partie rigide 41 et une partie rigide intermédiaire 44b, et une deuxième liaison flexible 44c selon l'axe y entre la partie rigide intermédiaire 44b et la seconde partie rigide 42. En d'autres termes, il s'agit de deux simples liaisons flexibles orientées chacune selon l'un des deux axes de rotations x et y. Cette liaison peut être nommée « double liaison flexible » ou « double liaison flexible à 2 degrés de liberté ».

Comme illustré en figure 8c, une liaison rotule ou sphérique (spherical joint) à trois degrés de liberté (rotations selon les axes x, y et z) est assurée par un système combinant une première liaison flexible 45a assurant une articulation selon l'axe y entre la première partie rigide 41 et une première partie rigide intermédiaire 45b, une deuxième liaison flexible 45c selon l'axe x entre la première partie rigide intermédiaire 45b et une seconde partie rigide intermédiaire 45d, et une troisième liaison flexible 45e selon l'axe z entre la seconde partie rigide intermédiaire 45d et la seconde partie rigide 42. En d'autres termes, il s'agit de trois simples liaisons flexibles orientées chacune selon l'un des trois axes de rotations x, y et z. Cette liaison peut être nommée « triple liaison flexible sphérique » ou « triple liaison flexible à 3 degrés de liberté ».

Ces trois types de liaisons remplacent avantageusement des liaisons conventionnelles, pour des dispositifs de dimensions millimétriques.

Le dispositif de micro-actionnement 100 selon l'invention met en œuvre trois liaisons flexibles configurées pour réaliser une articulation de type pivot (ou de type cardan) à un (ou à deux) degré(s) de liberté.

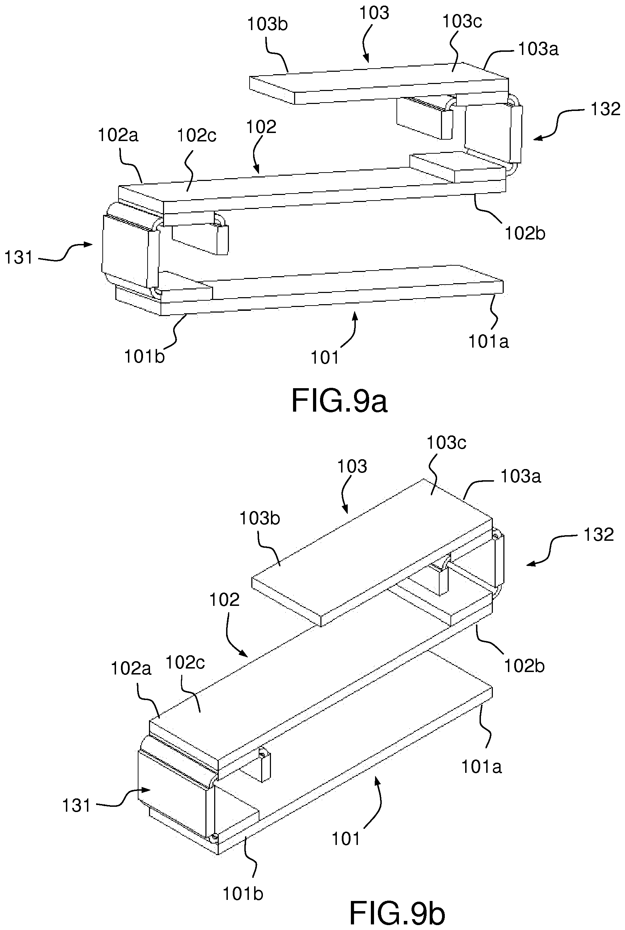

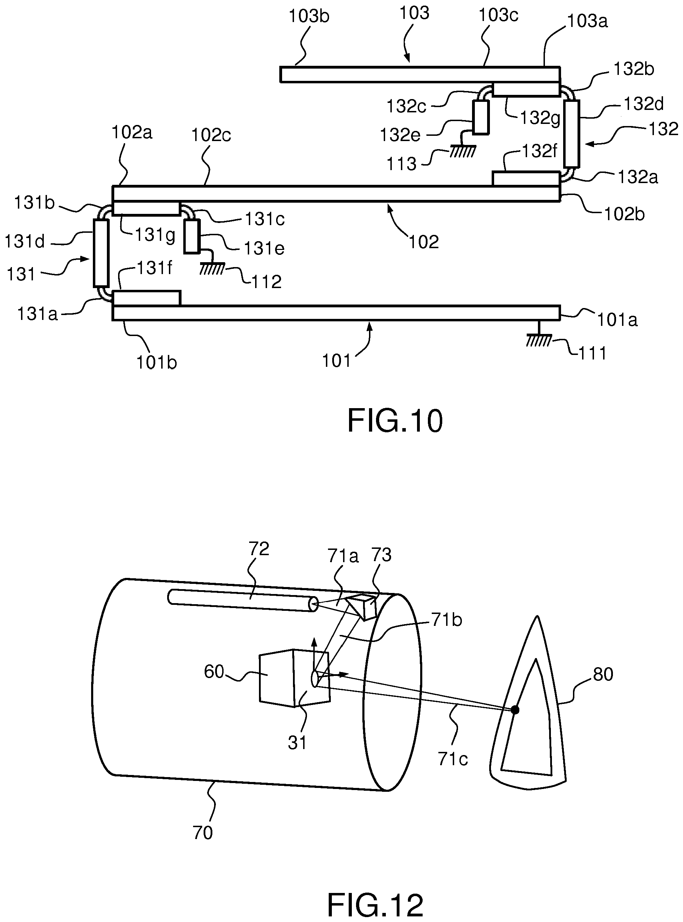

Les figures 9a et 9b illustrent un exemple d'un dispositif d'actionnement 100 comprenant trois poutres, vu en 3D selon deux angles différents. La figure 10 reprend le même exemple, en vue 2D. Elles représentent l'exemple d'un dispositif d'actionnement 100 illustré en vue cinématique sur les figures 3b et 4b, avec davantage de détails sur les liaisons flexibles qui ont été décrites avec les figures 5, 6a, 6b, 7a à 7c et 8a à 8e.

La première articulation 131 comprend plusieurs parties 131a, 131b, 131c, 131d, 131e, 131f et 131g qui sont les parties d'une pièce d'un seul tenant :

· ne première partie flexible 131a reliée à la première poutre 101 par l'intermédiaire d'une troisième partie rigide 131f,
· une deuxième partie flexible 131b reliée à la seconde poutre 102 par l'intermédiaire d'une quatrième partie rigide 131g,
· une première partie rigide 131d reliant les première et deuxième parties flexibles 131a et 131b,
· ne seconde partie rigide 131e pouvant être positionnée contre un second point fixe 112,
· une troisième partie flexible 131c reliant la seconde poutre 102 à la seconde partie rigide 131e au niveau du point de pivot 102c : ainsi l'ensemble formé par la seconde partie rigide 131e et la seconde poutre 102 forme un levier autour d'un point de pivot 102c de la deuxième poutre 102.

Les parties flexibles 131a, 131b et 131c sont des liaisons flexibles simples 40 qui sont toutes articulées autour d'axes parallèles entre eux et perpendiculaires à l'axe principal 124.

La seconde articulation 132 comprend plusieurs parties 132a, 132b, 132c, 132d, 132e, 132f, 132g qui sont les parties d'une pièce d'un seul tenant :

· une première partie flexible 132a reliée à la seconde poutre 102 par l'intermédiaire d'une troisième partie rigide 132f,
· une deuxième partie flexible 132b reliée à la troisième poutre 103 par l'intermédiaire d'une quatrième partie rigide 132g,
· une première partie rigide 132d reliant les première et deuxième parties flexibles 132a et 132b,
· une seconde partie rigide 132e pouvant être positionné contre un troisième point fixe 113,
· une troisième partie flexible 132c reliant la quatrième partie rigide 132g à la seconde partie rigide 132e au niveau du point de pivot 103c : ainsi l'ensemble formé par la seconde partie rigide 132e et la troisième poutre 103 forme un levier autour d'un point de pivot 103c de la troisième poutre 103.

Les parties flexibles 132a, 132b et 132c sont des liaisons flexibles simples 40 qui sont toutes articulées autour d'axes parallèles entre eux et perpendiculaires à l'axe principal 124.

Afin de finaliser la fabrication du dispositif d'actionnement 100 selon l'invention, les liaisons flexibles qui constituent une partie constitutive des articulations dans le dispositif d'actionnement sont assemblées aux poutres piézoélectriques.

Ainsi, dans l'exemple représenté :

· la première triple liaison 131 est reliée à la première poutre 101 en fixant, par exemple par collage ou par soudure, sa troisième partie rigide 131f au niveau de la seconde extrémité 101b de la première poutre et est reliée à la deuxième poutre 102 en fixant, par exemple par collage ou par soudure, sa quatrième partie rigide 131g au niveau de la première extrémité 102a de la seconde poutre.
· la deuxième triple liaison 132 est reliée à la deuxième poutre 102 en fixant, par exemple par collage ou par soudure, sa troisième partie rigide 132f au niveau de la seconde extrémité 102b de la deuxième poutre 102 et est reliée à la troisième poutre 103 en fixant, par exemple par collage ou par soudure, sa quatrième partie rigide 132g au niveau de la première extrémité 103a de la troisième poutre 103.

Ensuite, chaque poutre est connectée électriquement.

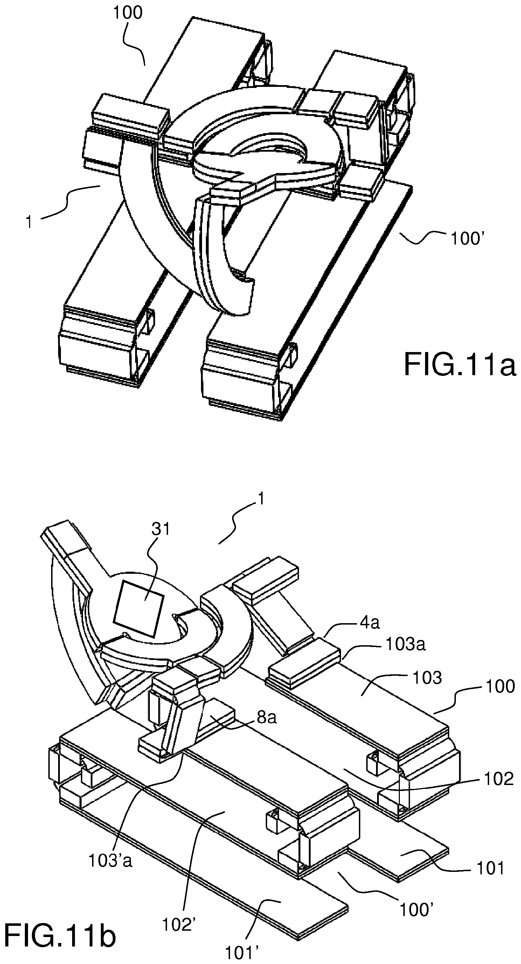

Les figures 11a, 11b illustrent un exemple de microrobot selon un autre aspect de l'invention, avant la mise en place du support et selon deux vues différentes, en 3D.

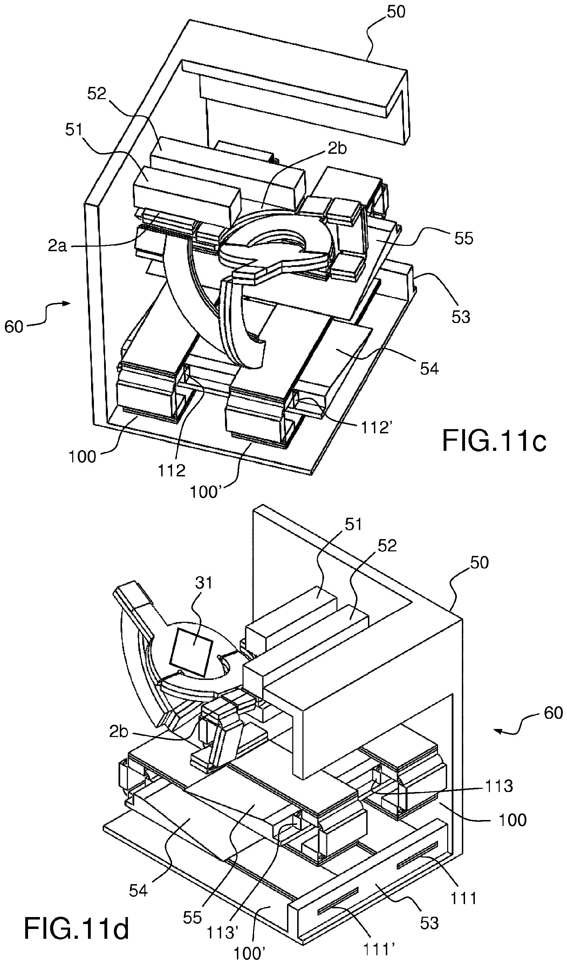

Les figures 11c, 11d illustrent un exemple de microrobot selon l'invention, avec son support et selon deux vues différentes, en 3D.

Un dispositif d'orientation sphérique 1 comprenant une plateforme 30 à orienter selon deux axes de rotation 22, 23 est combiné avec au moins deux dispositifs d'actionnement 100, 100' selon l'invention, un premier dispositif d'actionnement 100 permettant d'actionner le dispositif d'orientation 1 de manière à orienter la plateforme 30 selon un premier axe de rotation 22, et un second dispositif d'actionnement 100 permettant d'actionner le dispositif d'orientation 1 de manière à orienter la plateforme 30 selon un second axe de rotation 23.

Une telle combinaison reliant un dispositif d'orientation sphérique 1 et deux dispositifs d'actionnement 100, 100' selon l'invention permet de réaliser un microrobot à cinématique parallèle sphérique à deux degrés de liberté parallèle à deux degrés de liberté répondant aux contraintes attendues et présentées en introduction de la présente demande, notamment la précision d'orientation, tout en conservant les débattements des angles d'orientation souhaités.

Les dispositifs d'actionnement 100, 100' selon l'invention permettent en outre d'actionner à distance un dispositif d'orientation sphérique 1, puisqu'ils sont connectés électriquement, donc potentiellement à distance, et qu'ils agissent en flexion lorsqu'ils sont excités par une tension.

Dans l'exemple présenté dans les figures 11a, 11b, 11c, 11d, le dispositif d'orientation sphérique 1 comprend

- un premier bras d'actionnement 4, dont une première partie 4a est couplée avec un premier dispositif d'actionnement 100 selon l'invention, plus précisément avec la partie libre 103b correspondant à la seconde extrémité de la troisième poutre 103 dudit dispositif d'actionnement 100 ;
- et un second bras d'actionnement 8 dont une première partie 8a est couplée avec un second dispositif d'actionnement 100' selon l'invention plus précisément avec la partie libre 103'b correspondant à la seconde extrémité de la troisième poutre 103' dudit dispositif d'actionnement 100'.

Cela permet d'obtenir la structure illustrée en figures 11a et 11b.

Le point fixe 111 (resp 111') et les points fixes pivot 112, 113 (resp 112', 113') nécessaires au fonctionnement du dispositif d'actionnement 100 (resp 100') selon l'invention font partie du support 50. Ledit support 50 est également configuré pour créer les points fixes 2a et 2b pour le dispositif d'orientation sphérique 1, comme illustré en figures 11c et 11d.

Plus précisément, le support 50 comprend plusieurs parties 51, 52, 53, 54, 55, lesquelles parties étant configurées pour créer les parties fixes pour le microrobot 60. Par exemple :

- la partie 53 permet de créer les points fixes 111 et 111' pour les premières poutres 101 et 101' des dispositifs d'actionnement 100 et 100'
- la partie 54 permet de créer les points fixes pivot 112 et 112' pour les deuxièmes poutres 102 et 102' des dispositifs d'actionnement 100 et 100'
- et la partie 55 permet de créer les points fixes pivot 113 et 113' pour les troisièmes poutres 103 et 103' des dispositifs d'actionnement 100 et 100'
- les parties 51 et 52 étant configurées pour créer les points fixes 2a et 2b pour le système d'orientation sphérique 1.

Le microrobot 60 ainsi obtenu présente une structure d'architecture parallèle qui, contrairement à une structure d'architecture en série, peut mettre en œuvre des joints flexibles disposés à l'intérieur de ladite structure qui ne soient pas forcément actionnés, c'est-à-dire des joints passifs. Au contraire, les joints flexibles des structures en série doivent être tous munis d'un dispositif d'actionnement.

En outre, cela permet de gagner en volume. Ainsi, cette combinaison permet d'obtenir un microrobot dont les dimensions sont comprises dans un cube 10 x 10 x 10 mm³ au maximum, avec des détails sur le motif de la dizaine de μm.

Sur la plateforme 30 du dispositif d'orientation sphérique 1, un miroir 31 peut être positionné, comme illustré dans les figures 11a à 11d. Ainsi, le microrobot 60 permet d'orienter le miroir 31 selon les deux axes de rotation 22 et 23.

Des connexions électriques (non représentées sur les figures) nécessaires au pilotage à distance des dispositifs d'actionnement 100, 100' et au final à la rotation du miroir 31 sont ajoutées. Les connexions électriques peuvent être fixées sur le support 50, voire être incluses tout ou partie à l'intérieur du support, afin de minimiser le volume occupé par le microrobot 60 et ses connexions électriques.

Alternativement, d'autres structures cinématiques autres que sphériques peuvent être utilisées. En général toute structure cinématique qui puisse, au moins, orienter une plateforme sur deux axes coplanaires. Des structures parallèles et en série sont possibles.

Comme illustré en figure 12, le microrobot selon l'invention peut être utilisé, dans le domaine de la microchirurgie en tant que système terminal d'un endoscope souple 70. S'il s'agit du domaine spécifique de la phonochirurgie, l'endoscope est nommé larynscope.

Dans ce cas, le microrobot portant le miroir 31, en général un microrobot comprenant une plateforme, laquelle supporte un miroir, est utilisé pour orienter un faisceau laser 71. L'orientation initiale du miroir 31 doit être calculée lors de la conception géométrique du dispositif d'orientation 1 afin de refléter le faisceau laser 71 selon les directions et selon le débattement angulaire souhaité, correspondant à la zone à traiter 80.

Dans la configuration idéale, un faisceau laser 71a sera amené par une fibre optique 72 depuis l'extérieur jusqu'à l'extrémité distale de l'endoscope 70. Par exemple, la fibre optique génère un faisceau laser 71a qui est réfléchi par un prisme 73 pour renvoyer un faisceau laser 71b. Dans ce cas, le miroir 31 est disposé de manière à focaliser puis à refléter le faisceau laser 71b pour renvoyer un faisceau laser 71c. L'orientation du miroir plan est guidée par le microrobot. Ainsi, le microrobot 60 dirige le faisceau laser 71c sur les zones à traiter 80, par exemple sur les cordes vocales.

Le microrobot à cinématique parallèle sphérique à deux degrés de liberté 60 selon l'invention permet de répondre aux contraintes développées en introduction de la présenté invention, à savoir, pour une distance entre le miroir 31 et les cordes vocales de 20 mm :

- deux degrés de liberté au moins pour pouvoir intervenir sur l'ensemble de la zone à traiter 80 ;
- plage angulaire de $\pm$ 12,5° pour chaque axe de rotation du miroir 31 pour pouvoir intervenir sur l'ensemble de la zone à traiter 80 ;
- résolution de balayage du faisceau de 100 μm ou mieux, ce qui correspond à une résolution de 0,15° pour chaque angle de rotation du miroir 31 à la distance indiquée de 20 mm, ce pour ne pas risquer d'abimer des cellules saines en traitant des cellules malignes. ;

- bande passante de déplacement du faisceau de 200Hz au moins ;
- biocompatibilité de l'ensemble des composants du microrobot 60 ;
- volume occupé par le microrobot 60 inférieur à 10 x 10 x 10 mm$^3$.

En conclusion le microrobot selon l'invention permet de répondre au besoin d'un dispositif de petite taille (qui puisse entrer dans la gorge), pouvant faire balayer un faisceau laser selon une plage angulaire définie et selon deux degrés de liberté, avec une grande vitesse (pour ne pas rester longtemps sur une position) et une résolution assez fine (pour distinguer les cellules saines des cellules malignes).

Avantageusement, le microrobot permet d'accueillir au moins un système de visualisation, par exemple une caméra, afin de vérifier la position du faisceau laser, de préférence le faisceau laser 71b, avant qu'il n'atteigne la zone 80 à traiter.

L'invention peut trouver de nombreuses applications, notamment pour actionner une plateforme, ladite plateforme pouvant accueillir un miroir pour réfléchir un faisceau laser. Le faisceau laser pour être ainsi utilisé pour le marquage et/ou la gravure laser, pour le scan 3D, dans le domaine de la (micro)robotique (vision par laser par exemple), dans le domaine des télécommunications (par exemple pour un atténuateur optique variable ou un commutateur optique), ou pour des applications médicales (chirurgie endoscopique peu invasive, exploration optique avec ou sans biopsie ...).

Le microrobot selon l'invention peut être utilisé sur des drones, des véhicules autonomes et plus généralement sur de la robotique mobile.

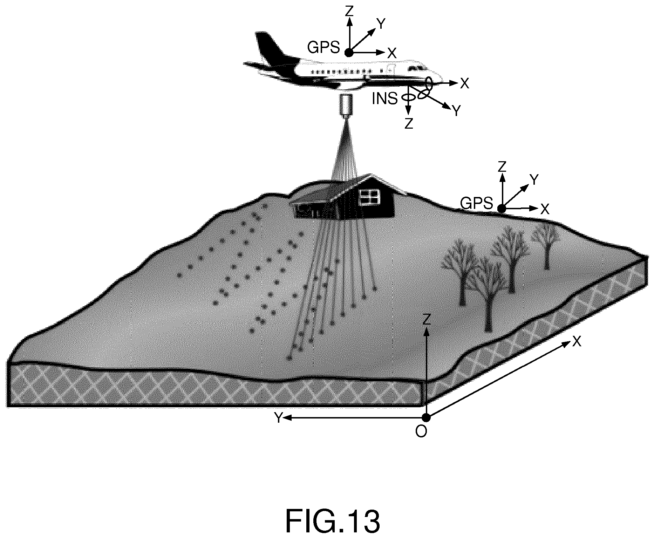

En particulier, comme illustré en figure 13, une application du microrobot est de miniaturiser et de rendre mobiles les dispositifs de scan pour LIDAR (Light Détection and Ranging). Sur un LIDAR, un dispositif réalise un balayage d'un laser sur la surface d'un terrain pour caractériser son relief. La distance et le profil des éléments qui figurent sur la surface du terrain (maisons, arbres, voitures, eau, ...) est calculée par interférométrie. Le microrobot selon l'invention peut avantageusement réaliser un tel balayage.

**Revendications**

1. Dispositif d'actionnement amplificateur de mouvement (100), **caractérisé en ce qu'**il comprend :

    - une première poutre (101) comprenant un élément piézoélectrique (P), étant apte à fléchir selon un axe principal (124) lorsqu'une tension lui est appliquée et apte à être attachée en une première extrémité (101a) à un premier point fixe (111) ;
- une seconde poutre (102) comprenant un élément piézoélectrique, apte à fléchir selon ledit axe principal (124) lorsqu'une tension lui est appliquée, et présentant une première extrémité (102a) et une seconde extrémité (102b);
- une première articulation (131) comprenant :

    une première partie flexible (131a) selon un axe perpendiculaire à l'axe principal (124) et reliée à la première poutre (101) en la seconde extrémité (101b) de ladite première poutre,
    une deuxième partie flexible (131b) selon un axe perpendiculaire à l'axe principal (124) et reliée à la seconde poutre (102) en la première extrémité (102a) de ladite seconde poutre,
    une première partie rigide (131d) reliant les première et deuxième parties flexibles (131a, 131b),
    une seconde partie rigide (131e) apte à être positionnée contre un deuxième point fixe (112),
    une troisième partie flexible (131c) selon un axe perpendiculaire à l'axe principal (124) reliant la seconde poutre (102) à la seconde partie rigide (131e) en un point de pivot (102c) de ladite seconde poutre (102), de sorte que l'ensemble formé par la seconde partie rigide (131e) et la seconde poutre (102) forme un levier autour dudit point de pivot (102c), lesdites parties flexibles (131a, 131b, 131c) et rigides (131d, 131e) étant les parties d'une pièce d'un seul tenant.

2. Dispositif d'actionnement amplificateur de mouvement (100) selon la revendication 1 comprenant, en outre :

    - une troisième poutre (103) comprenant un élément piézo-électrique (P), étant apte à fléchir selon l'axe principal (124) lorsqu'une tension lui est appliquée, et présentant une première extrémité (103a) et une seconde extrémité (103b) ;
    - une seconde articulation (132) comprenant :

    une première partie flexible (132a) selon un axe perpendiculaire à l'axe principal (124) et reliée à la seconde poutre (102) en la seconde extrémité (102b) de ladite seconde poutre,
    une deuxième partie flexible (132b) selon un axe perpendiculaire à l'axe principal (124) et reliée à la troisième poutre (103) en la première extrémité (103a) de ladite troisième poutre,

■ une première partie rigide (132d) reliant les première et deuxième parties flexibles (132a, 132b),

■ une seconde partie rigide (132e) apte à être positionnée contre un troisième point fixe (113),

■ une troisième partie flexible (132c) selon un axe perpendiculaire à l'axe principal (124) reliant la troisième poutre (103) à la seconde partie rigide (132e) en un point de pivot (103c) de ladite troisième poutre (103), de sorte que l'ensemble formé par la seconde partie rigide (132e) et la seconde poutre (132) forme un levier autour dudit point de pivot (103c), lesdites parties flexibles (132a, 132b, 132c) et rigides (132d, 132e) étant les parties d'une pièce d'un seul tenant.

3. Dispositif d'actionnement amplificateur de mouvement (100) selon la revendication 1 ou 2 comprenant :

- une première poutre (101) comprenant un élément piézoélectrique (P), étant apte à fléchir selon un axe principal (124) lorsqu'une tension lui est appliquée, et apte à être attachée en une première extrémité (101a) à un premier point fixe (111) ;
- N autres poutres (10M), N étant supérieur ou égal à 2 et M variant entre 2 et N, chaque M-ième poutre comprenant un élément piézo-électrique (P), étant apte à fléchir selon l'axe principal (124) lorsqu'une tension lui est appliquée, et présentant une première extrémité (10Ma) et une seconde extrémité (10Mb) ; la N-ième poutre (10N) présentant une seconde extrémité (10Nb) libre ;
- X articulations (13Y), X étant égal à N-1 et Y étant égal à M-1, chaque Y-ième articulation (13Y) comprenant :

une première partie flexible (13Ya) selon un axe perpendiculaire à l'axe principal (124) et reliée à la Y-ième poutre (10Y) en la seconde extrémité (10Yb) de ladite Y-ième poutre,

une deuxième partie flexible (13Yb) selon un axe perpendiculaire à l'axe principal (124) et reliée à la M-ième poutre (10M) en la première extrémité (10Ma) de ladite M-ième poutre,

une première partie rigide (13Yd) reliant les première et deuxième parties flexibles,

une seconde partie rigide (13Ye) apte à être positionnée contre un M-ième point fixe (11M),

une troisième partie flexible (13Yc) selon un axe perpendiculaire à l'axe principal (124) reliant la M-ième poutre (10M) à la seconde partie rigide (13Ye) en un point de pivot (10Mc) de ladite M-ième poutre (10M), de sorte que l'ensemble formé par la seconde partie rigide (13Ye) et la M-ième poutre (13M) forme un levier autour dudit point de pivot (10Mc) ;

lesdites parties flexibles (13Ya, 13Yb, 13Yc) et rigides (13Yd, 13Ye) étant les parties d'une pièce d'un seul tenant.

4. Dispositif d'actionnement amplificateur de mouvement (100) selon la revendication 3, dans lequel au moins une Y-ième articulation (13Y) comprend, en outre, une troisième (13Yf) et une quatrième (13Yg) parties rigides formant avec les autres parties de ladite articulation une pièce d'un seul tenant :

- la troisième partie rigide (13Yf) formant la liaison entre la première partie flexible (13Ya) de ladite Y-ième articulation (13Y) avec la Y-ième poutre (10Y),
- la quatrième partie rigide (13Yg) formant la liaison entre la deuxième flexible (13Yb) de ladite de ladite Y-ième articulation (13Y) avec la M-ième poutre (10M).

5. Dispositif d'actionnement amplificateur de mouvement (100) selon l'une quelconque des revendications précédentes, dans lequel les parties flexibles d'au moins une Y-ième articulation (13Y) présentent des axes d'articulation parallèles entre eux et perpendiculaires à l'axe principal (124).

6. Dispositif d'actionnement amplificateur de mouvement (100) selon l'une quelconque des revendications précédentes, dans lequel l'élément piézoélectrique (P) comprend du titanate de zirconate au plomb.

7. Dispositif d'actionnement amplificateur de mouvement (100) selon l'une quelconque des revendications précédentes, dans lequel au moins une poutre (10M) présente une structure bimorphe.

8. Microrobot (60) à cinématique parallèle sphérique à deux degrés de liberté comprenant :

- un dispositif d'orientation à deux degrés de liberté comprenant une plateforme (30) à orienter selon un premier (22) et un second (23) axes de rotation par rapport à une base fixe (2), un premier bras d'actionnement (4) et un second bras d'actionnement (8) ;
- un premier et un deuxième dispositifs d'actionnement amplificateur de mouvement (100, 100') selon l'une quelconque des revendications 1 à 7 ;

le premier dispositif d'actionnement (100) étant connecté au premier bras d'actionnement (4) de manière à lui transmettre un premier mouvement de translation relativement à la base fixe (2), de sorte à entrainer la plateforme (30) en rotation selon le premier axe (22), et
le deuxième dispositif d'actionnement (100') étant connecté au second bras d'actionnement (8) de manière à lui transmettre un second mouvement de translation relativement à la base fixe (2) de sorte à entrainer la plateforme (30) en rotation selon le second axe (23).

9. Microrobot (60) selon la revendication 8, dans lequel le dispositif d'orientation est un dispositif d'orientation sphérique (1) à deux degrés de liberté reliant la plateforme (30) à deux points de fixation (2a, 2b) d'une base fixe (2) de manière à pouvoir orienter ladite plateforme (30) dans l'espace par rotation autour d'un premier axe (22) et d'un second axe (23), ces deux axes (22, 23) étant sensiblement perpendiculaires et se croisant en un centre de mouvement sphérique (21) situé dans ledit organe (30) à orienter, comprenant :

- un premier bras d'actionnement (4) configuré pour effectuer un mouvement de translation relativement à la base fixe (2) et apte à appliquer à un premier bras de transmission (5), relié à un premier point de fixation (2a) de la base fixe (2) par une liaison flexible (6) articulée selon le premier axe (22), un mouvement de rotation relativement à ladite base fixe (2) de manière à transmettre à la plateforme (30) un mouvement de rotation selon ledit premier axe (22) ;
- un bras intermédiaire (12) relié au premier bras de transmission (5) par une liaison flexible (13) articulée autour d'un troisième axe (24), perpendiculaire aux premier et second axes (22) et (23), et relié à la plateforme (30) par une liaison flexible (14), de manière à transmettre à la plateforme (30) un mouvement de rotation selon le premier axe (22) ;
- un second bras d'actionnement (8) configuré pour effectuer un mouvement de translation relativement à la base fixe (2) et apte à appliquer à un second bras de transmission (9), relié à un second point de fixation (2b) de la base fixe (2) par une liaison flexible (10) articulée selon le second axe (23), un mouvement de rotation relativement à ladite base fixe (2) de manière à appliquer sur la plateforme (30) un mouvement de rotation selon ledit second axe (23), ledit bras (9) étant relié à la plateforme (30) par une liaison flexible (15, 16) articulée selon le premier axe (22), de manière à ne pas entrainer ledit second bras de transmission (9) en rotation selon le premier axe (22) lors de l'actionnement du premier bras d'actionnement (4) ;

la liaison (14) entre la plateforme (30) et le bras intermédiaire (12) étant articulée selon l'axe (23) de manière à ne pas entrainer ledit bras intermédiaire (12) en rotation selon le second axe (23) lors de l'actionnement du second bras d'actionnement (8) ;
et les bras (4, 5, 8, 9, 12), les liaisons flexibles (6, 10, 13, 14, 15, 16) et la plateforme (30) formant les parties d'une pièce formée en un seul tenant formant le dispositif (1), les bras (4, 5, 8, 9, 12) et la plateforme (30) étant des parties rigides du dispositif (1), et les liaisons flexibles (6, 10, 13, 14, 15, 16) étant des parties flexibles formant chacune une charnière selon un seul des premier, second et troisième axes (22, 23, 24), et reliant lesdites parties rigides entre elles, à la base fixe (2).

10. Microrobot (60) selon la revendication 8 ou 9, dans lequel les parties rigides (4, 5, 8, 9, 12, 30) comprennent une couche centrale (41) d'un matériau flexible, tel un polyimide, prise en sandwich entre deux couches (42, 43) d'un matériau rigide, telle de la fibre de carbone, les liaisons flexibles (6, 10, 13, 14, 15, 16) étant composées de la couche centrale.

11. Microrobot (60) selon l'une quelconque des revendications 8 à 10, dans lequel un ou des bras de transmission (5, 9) et/ou bras intermédiaire (12) forment un arc de cercle.

12. Microrobot (60) selon l'une quelconque des revendications 8 à 11, dans lequel le dispositif d'orientation sphérique (1) comprend, en outre, une liaison flexible (7) articulée autour d'un axe parallèle au premier axe (22) et disposée entre le premier bras d'actionnement (4) et le premier bras de transmission (5), et formant avec les bras (4, 5, 8, 9, 12), les liaisons flexibles (6, 10, 13, 14, 15, 16) et la plateforme (30) une pièce d'un seul tenant

13. Microrobot (60) selon l'une quelconque des revendications 8 à 12, le premier bras d'actionnement (4) comprenant une première partie (4a) apte à être couplée au dispositif d'actionnement (100) et une seconde partie (4b) reliée au premier bras de transmission (5) par une liaison flexible (7) articulée autour d'un axe parallèle au premier axe (22), les lesdites parties première et seconde parties (4a) et (4b) étant reliées par une liaison flexible (17) articulée selon un axe parallèle au premier axe (22) et formant avec les bras (4, 5, 8, 9, 12), les liaisons flexibles (6, 7, 10, 13, 14, 15, 16) et la plateforme (30) une pièce d'un seul tenant.

14. Microrobot (60) selon l'une quelconque des revendications 8 à 13, le dispositif d'orientation sphérique (1) comprenant, en outre, une liaison flexible (11) reliant le second bras d'actionnement (8) et le second bras de transmission (9), articulée autour d'un axe parallèle au second axe (23) et formant avec les bras (4, 5, 8, 9, 12), les liaisons flexibles (6, 10, 13, 14, 15, 16) et la plateforme (30) une pièce d'un seul tenant.

15. Microrobot (60) selon l'une quelconque des revendications 8 à 14, dans lequel le dispositif d'orientation sphérique (1) selon l'une quelconque des revendications 1 à 5 comprend, en outre, une double liaison flexible (19) de type cardan reliant le second bras d'actionnement (8) et le second bras de transmission (9), articulée autour d'un axe parallèle au premier axe (22) et d'un axe parallèle au second axe (23), et formant avec les bras (4, 5, 8, 9, 12), les liaisons flexibles (6, 10, 13, 14, 15, 16) et la plateforme (30) une pièce d'un seul tenant.

16. Microrobot (60) selon l'une quelconque des revendications 8 à 15, dans lequel le second bras d'actionnement (8) comprend une première partie (8a) apte à être couplée au dispositif d'actionnement (100') et une seconde partie (8b) reliée au second bras de transmission (9) par une simple liaison flexible simple (11) ou une double liaison flexible (19), les première et seconde parties (8a) et (8b) étant reliées une double liaison flexible (18) de type cardan articulée autour d'un axe parallèle au premier axe (22) et d'un axe parallèle au second axe (23), et formant avec les bras (4, 5, 8, 9, 12), les liaisons flexibles (6, 10, 13, 14, 15, 16) et la plateforme (30) une pièce d'un seul tenant.

17. Microrobot (60) selon l'une quelconque des revendications 8 à 16 comprenant, en outre, un support (50) configuré pour créer les points fixes des dispositifs d'actionnement (100, 100').

18. Microrobot (60) selon l'une quelconque des revendications 8 à 17, dans lequel la plateforme (30) supporte un miroir (31).

19. Microrobot (60) selon la revendication 18, dans lequel le miroir (31) est disposé de manière à réfléchir un faisceau laser (71).

20. Microrobot (60) selon la revendication 19 comprenant, en outre, un dispositif de visualisation d'un spot du faisceau laser (71) positionné sur une surface.

**Patentansprüche**

1. Bewegungsverstärkende Betätigungsvorrichtung (100), **dadurch gekennzeichnet, dass** sie aufweist:

- einen ersten Träger (101), der ein piezoelektrisches Element (P) aufweist, das in der Lage ist, sich entlang einer Hauptachse (124) zu biegen, wenn Spannung darauf aufgebracht wird, und der geeignet ist, an einem ersten Ende (101a) an einem Festpunkt (111) angebracht zu werden;
- einen zweiten Träger (102), der ein piezoelektrisches Element aufweist, das in der Lage ist, sich entlang der Hauptachse (124) zu biegen, wenn Spannung darauf aufgebracht wird, und der ein erstes Ende (102a) und ein zweites Ende (102b) aufweist;
- ein erstes Gelenk (131), welches aufweist:

  - ein erstes Teil (131a), das entlang einer zu der Hauptachse (124) senkrechten Achse flexibel ist und an dem zweiten Ende (101b) des ersten Trägers mit dem ersten Träger (101) verbunden ist,
  - ein zweites Teil (131b), das entlang einer zu der Hauptachse (124) senkrechten Achse flexibel ist und an dem ersten Ende (102a) des zweiten Trägers mit dem zweiten Träger (102) verbunden ist,
  - ein erstes starres Teil (131d), welches das erste und das zweite flexible Teil (131a, 131b) miteinander verbindet,
  - ein zweites starres Teil (131e), das gegen einen zweiten Festpunkt (112) positionierbar ist,
  - ein entlang einer zu der Hauptachse (124) senkrechten Achse flexibles drittes Teil (131c), das den zweiten Träger (102) mit dem zweiten starren Teil (131e) an einem Schwenkpunkt (102c) des zweiten Trägers (102) derart verbindet, dass die von dem zweiten starren Teil (131e) und dem zweiten Träger (102) gebildete Einheit einen Hebel um den Schwenkpunkt (102c) bildet, wobei die flexiblen (131a, 131b, 131c) und die starren Teile (131d, 131e) die Bestandteile eines einstückigen Teils sind.

2. Bewegungsverstärkende Betätigungsvorrichtung (100) nach Anspruch 1, ferner aufweisend:

- einen dritten Träger (103), der ein piezoelektrisches Element (P) aufweist, das in der Lage ist, sich entlang der Hauptachse (124) zu biegen, wenn Spannung darauf aufgebracht wird, und der ein erstes Ende (193a) und ein zweites Ende (103b) aufweist;
- ein zweites Gelenk (132), welches aufweist:

  - ein erstes Teil (132a), das entlang einer zu der Hauptachse (124) senkrechten Achse flexibel ist und an dem zweiten Ende (102b) des zweiten Trägers mit dem zweiten Träger (102) verbunden ist,

- ein zweites Teil (132b), das entlang einer zu der Hauptachse (124) senkrechten Achse flexibel ist und an dem ersten Ende (103a) des dritten Trägers mit dem dritten Träger (103) verbunden ist,

- ein erstes starres Teil (132d), welches das erste und das zweite flexible Teil (132a, 132b) miteinander verbindet,

- ein zweites starres Teil (132e), das gegen einen dritten Festpunkt (113) positionierbar ist,

- ein entlang einer zu der Hauptachse (124) senkrechten Achse flexibles drittes Teil (132c), das den dritten Träger (103) mit dem zweiten starren Teil (132e) an einem Schwenkpunkt (103c) des dritten Trägers (102) derart verbindet, dass die von dem zweiten starren Teil (132e) und dem zweiten Träger (132) gebildete Einheit einen Hebel um den Schwenkpunkt (103c) bildet, wobei die flexiblen (132a, 132b, 132c) und die starren Teile (132d, 132e) die Bestandteile eines einstückigen Teils sind.

3. Bewegungsverstärkende Betätigungsvorrichtung (100) nach Anspruch 1 oder 2, welche aufweist:

- einen ersten Träger (101), der ein piezoelektrisches Element (P) aufweist, das in der Lage ist, sich entlang einer Hauptachse (124) zu biegen, wenn Spannung darauf aufgebracht wird, und der geeignet ist, an einem ersten Ende (101a) an einem Festpunkt (111) angebracht zu werden;

- N weitere Träger (10M), wobei N größer oder gleich 2 ist und M zwischen 2 und N variiert, wobei jeder M-te Träger ein piezoelektrisches Element (P), das in der Lage ist, sich entlang einer Hauptachse (124) zu biegen, wenn Spannung darauf aufgebracht wird, und ein erstes Ende (10Ma) und ein zweites Ende (10Mb) aufweist; wobei der N-te Träger (10N) ein freies zweites Ende (10Nb) aufweist;

- X Gelenke (13Y), wobei X gleich N-1 und Y gleich M-1 ist, wobei jedes Y-te Gelenk (13Y) aufweist:

- ein erstes Teil (13Ya), das entlang einer zu der Hauptachse (124) senkrechten Achse flexibel ist und an dem zweiten Ende (10Yb) des Y-ten Trägers mit dem Y-ten Träger (10Y) verbunden ist,

- ein zweites Teil (13Yb), das entlang einer zu der Hauptachse (124) senkrechten Achse flexibel ist und an dem M-ten Ende (10Ma) des M-ten Trägers mit dem M-ten Träger (10M) verbunden ist,

- ein erstes starres Teil (13Yd), welches das erste und das zweite flexible Teil miteinander verbindet,

- ein zweites starres Teil (13Ye), das gegen einen M-ten Festpunkt (11M) positionierbar ist,

- ein entlang einer zu der Hauptachse (124) senkrechten Achse flexibles drittes Teil (13Yc), das den M-ten Träger (10M) mit dem zweiten starren Teil (13Ye) an einem Schwenkpunkt (10Mc) des M-ten Trägers (10M) derart verbindet, dass die von dem zweiten starren Teil (13Ye) und dem M-ten Träger (13M) gebildete Einheit einen Hebel um den Schwenkpunkt (10Mc) bildet, wobei die flexiblen (13Ya, 13Yb, 13Yc) und die starren Teile (13Yd, 13Ye) die Bestandteile eines einstückigen Teils sind.

4. Bewegungsverstärkende Betätigungsvorrichtung (100) nach Anspruch 3, bei welcher mindestens ein Y-tes Gelenk (13Y) ferner ein drittes (13Yf) und ein viertes starres Teil (13Yg) aufweist, welche mit den anderen Teilen des Gelenks ein einstückiges Teil bilden:

- wobei das dritte starre Teil (13Yf) die Verbindung zwischen dem ersten flexiblen Teil (13Ya) des Y-ten Gelenks (13Y) und dem Y-ten Träger (10Y) bildet,

- wobei das vierte starre Teil (13Yg) die Verbindung zwischen dem zweiten flexiblen Teil (13Yb) des Y-ten Gelenks (13Y) und dem M-ten Träger (10M) bildet.

5. Bewegungsverstärkende Betätigungsvorrichtung (100) nach einem der vorangehenden Ansprüche, bei welcher die flexiblen Teile mindestens eines Y-ten Gelenks (13Y) zueinander parallele und zu der Hauptachse (124) senkrechte Gelenkachsen aufweisen.

6. Bewegungsverstärkende Betätigungsvorrichtung (100) nach einem der vorangehenden Ansprüche, bei welcher das piezoelektrische Element (P) Titanat-Bleizirkonat aufweist.

7. Bewegungsverstärkende Betätigungsvorrichtung (100) nach einem der vorangehenden Ansprüche, bei welcher mindestens ein Träger (10M) eine bimorphe Struktur aufweist.

8. Mikroroboter (60) mit paralleler sphärischer Kinematik mit zwei Freiheitsgraden, welcher aufweist:

- eine Ausrichtvorrichtung mit zwei Freiheitsgraden, welche eine entlang einer ersten (22) und einer zweiten Drehachse (23) in Bezug auf eine feste Basis (2) ausrichtbare Plattform (30), einen ersten Betätigungsarm (4) und einen zweiten Betätigungsarm (8) aufweist;

- eine erste und eine zweite bewegungsverstärkende Betätigungsvorrichtung (100, 100') nach einem der Ansprüche 1 bis 7;

wobei die erste Betätigungsvorrichtung (100) mit dem ersten Betätigungsarm (4) derart verbunden ist, dass sie an diesen eine erste Translationsverschiebung in Bezug auf die feste Basis (2) überträgt, um die Plattform (30) entlang der ersten Achse (22) drehend anzutreiben, und

wobei die zweite Betätigungsvorrichtung (100') mit dem zweiten Betätigungsarm (8) derart verbunden ist, dass sie an diesen eine zweite Translationsverschiebung in Bezug auf die feste Basis (2) überträgt, um die Plattform (30) entlang der zweiten Achse (23) drehend anzutreiben.

**9.** Mikroroboter (60) nach Anspruch 8, bei welchem die Ausrichtvorrichtung eine sphärische Ausrichtvorrichtung (1) mit zwei Freiheitsgraden ist, welche die Plattform (30) mit zwei Befestigungspunkten (2a, 2b) einer festen Basis (2) derart verbindet, dass sie die Plattform (30) durch Drehen um eine erste Achse (22) und eine zweite Achse (23) im Raum ausrichten kann, wobei die beiden Achsen (22, 23) im Wesentlichen senkrecht sind und sich in einem sphärischen Bewegungsmittelpunkt (21) kreuzen, der sich in dem auszurichtenden Organ (30) befindet, aufweisend:

  - einen ersten Betätigungsarm (4), der dazu ausgebildet ist, eine Translationsbewegung in Bezug auf die feste Basis (2) durchzuführen und dazu geeignet ist, auf einen ersten Übertragungsarm (5), der mit einem ersten Befestigungspunkt (2a) der festen Basis (2) durch eine entlang der ersten Achse (22) gelenkige flexible Verbindung (6) verbunden ist, eine Drehbewegung in Bezug auf die feste Basis (2) zu übertragen, um an die Plattform (30) eine Drehbewegung entlang der ersten Achse (22) zu übertragen;

  - einen Mittelarm (12), der mit dem ersten Übertragungsarm (5) durch eine flexible um eine zu der ersten und der zweiten Achse (22) und (23) senkrechte dritte Achse (24) gelenkige Verbindung (13) verbunden ist, und mit der Plattform (30) durch eine flexible Verbindung (14) verbunden ist, um an die Plattform (30) eine Drehbewegung entlang der ersten Achse (22) zu übertragen;

  - einen zweiten Betätigungsarm (8), der dazu ausgebildet ist, eine Translationsbewegung in Bezug auf die feste Basis (2) durchzuführen und dazu geeignet ist, auf einen zweiten Übertragungsarm (9), der mit einem zweiten Befestigungspunkt (2b) der festen Basis (2) durch eine entlang der zweiten Achse (23) gelenkige flexible Verbindung (10) verbunden ist, eine Drehbewegung in Bezug auf die feste Basis (2) zu übertragen, um an die Plattform (30) eine Drehbewegung entlang der zweiten Achse (23) zu übertragen, wobei der Arm (9) mit der Plattform (30) durch eine entlang der ersten Achse (22) gelenkige flexible Verbindung (15, 16) derart verbunden ist, dass der zweite Übertragungsarm (9) bei der Betätigung des ersten Betätigungsarms (4) nicht entlang der ersten Achse (22) drehend angetrieben wird;

wobei die Verbindung (14) zwischen der Plattform (30) und dem Zwischenarm (12) derart entlang der Achse gelenkig ist, dass der Zwischenarm (12) bei der Betätigung des zweiten Betätigungsarms (8) nicht entlang der zweiten Achse (23) drehend angetrieben wird;

und die Arme (4, 5, 8, 9, 12), die flexiblen Verbindungen (6, 10, 13, 14, 15, 16) und die Plattform (30) die Bestandteile eines einstückigen Teils bilden, welches die Vorrichtung bildet, wobei die Arme (4, 5, 8, 9, 12) und die Plattform (30) starre Teile der Vorrichtung (1) sind und die flexiblen Verbindungen (6, 10, 13, 14, 15, 16) flexible Teile sind, die jeweils ein Scharnier entlang einer einzelnen der ersten, zweiten und dritten Achsen (22, 23, 24) bilden und die starren Teile untereinander, mit der festen Basis (2) verbinden.

**10.** Mikroroboter (60) nach Anspruch 8 oder 9, bei welchem die starren Teile (4, 5, 8, 9, 12, 30) eine mittlere Schicht (41) aus einem flexiblen Material, wie Polyimid, aufweisen, die sandwichartig zwischen zwei Schichten (42, 43) aus einem starren Material, wie Carbonfaser, gehalten ist, wobei die flexiblen Verbindungen (6, 10, 13, 14, 15, 16) aus der mittleren Schicht zusammengesetzt sind.

**11.** Mikroroboter (60) nach einem der Ansprüche 8 bis 10, bei welchem ein Übertragungsarm oder -arme (5, 9) und/oder ein Mittelarm (12) einen Kreisbogen bilden.

**12.** Mikroroboter (60) nach einem der Ansprüche 8 bis 11, bei welchem die sphärische Ausrichtvorrichtung (1) ferner eine flexible Verbindung (7) aufweist, die um eine zu der ersten Achse (22) parallele Achse gelenkig ist und zwischen dem ersten Betätigungsarm (4) und dem ersten Übertragungsarm (5) angeordnet ist, und mit den Armen (4, 5, 8, 9, 12), den flexiblen Verbindungen (6, 10, 13, 14, 15, 16) und der Plattform (30) ein einstückiges Teil bildet.

**13.** Mikroroboter (60) nach einem der Ansprüche 8 bis 12, bei welchem der erste Betätigungsarm (4) ein mit der Betätigungsvorrichtung (100) koppelbares erstes Teil (4a) und ein zweites Teil (4b) aufweist, das mit dem ersten Übertragungsarm (5) durch eine um eine zu der ersten Achse (22) parallele Achse gelenkige flexible Verbindung (7) verbunden ist, wobei das erste und das zweite Teil (4a) und (4b) durch eine entlang einer zu der ersten Achse (22) parallelen Achse gelenkige flexible Verbindung verbunden sind, und mit den Armen (4, 5, 8, 9, 12), den flexiblen Verbindungen (6, 10, 13, 14, 15, 16) und der Plattform (30) ein einstückiges Teil bilden.

**14.** Mikroroboter (60) nach einem der Ansprüche 8 bis 13, bei welchem die sphärische Ausrichtvorrichtung (1) ferner eine flexible Verbindung (11) aufweist, welche den zweiten Betätigungsarm (8) und den zweiten Betätigungsarm (9) miteinander verbindet, um eine zu der zweiten Achse (23) parallele Achse gelenkig ist, und mit den Armen (4, 5, 8, 9, 12), den flexiblen Verbindungen (6, 10, 13, 14, 15, 16) und der Plattform (30) ein einstückiges Teil bildet.

**15.** Mikroroboter (60) nach einem der Ansprüche 8 bis 14, bei welchem die sphärische Ausrichtvorrichtung /1) nach einem der Ansprüche 1 bis 5 ferner eine doppelte flexible Verbindung (19) vom Kardantyp aufweist, welche den zweiten Betätigungsarm (8) und den zweiten Übertragungsarm (9) verbindet, um eine zu der ersten Achse (22) parallel Achse und eine zu der zweiten Achse (23) parallele Achse gelenkig ist, und mit den Armen (4, 5, 8, 9, 12), den flexiblen Verbindungen (6, 10, 13, 14, 15, 16) und der Plattform (30) ein einstückiges Teil bildet.

**16.** Mikroroboter (60) nach einem der Ansprüche 8 bis 15, bei welchem der zweite Betätigungsarm (8) ein mit dem Betätigungselement (100') koppelbares erstes Teil (8a) und ein zweites Teil (8b) aufweist, das mit dem zweiten Übertragungsarm (9) durch eine einfache flexible Verbindung (11) oder eine doppelte flexible Verbindung (19) verbunden ist, wobei das erste und das zweite Teil (8a) und (8b) durch eine doppelte flexible Verbindung (18) vom Kardantyp verbunden sind, die um eine zu der ersten Achse (22) parallele Achse und eine zu der zweiten Achse (23) parallele Achse gelenkig ist, und mit den Armen (4, 5, 8, 9, 12), den flexiblen Verbindungen (6, 10, 13, 14, 15, 16) und der Plattform (30) ein einstückiges Teil bilden.

**17.** Mikroroboter (60) nach einem der Ansprüche 8 bis 16, welcher ferner eine Stütze (50) aufweist, die dazu ausgebildet ist, Festpunkte der Betätigungsvorrichtungen (100, 100') zu bilden.

**18.** Mikroroboter (60) nach einem der Ansprüche 8 bis 17, bei welchem die Plattform (30) einen Spiegel (31) stützt.

**19.** Mikroroboter (60) nach Anspruch 18, bei welchem der Spiegel (31) derart angeordnet ist, einen Laserstrahl (71) zu reflektieren.

**20.** Mikroroboter (60) nach Anspruch 19, welcher ferner eine Vorrichtung zur Visualisierung eines Punkts des Laserstrahls (71) aufweist, die auf einer Fläche positioniert ist.

**Claims**

**1.** A movement amplifying actuation device (100), **characterized in that** it comprises:

- a first beam (101) comprising a piezoelectric element (P), adapted to flex about a principal axis (124) when a voltage is applied to it and adapted to be attached at a first end (101a) to a first fixed point (111);
- a second beam (102) comprising a piezoelectric element, adapted to flex about said principal axis (124) when a voltage is applied to it, and having a first end (102a) and a second end (102b);
- a first articulation (131) comprising:

a first portion (131a) flexible about an axis perpendicular to the principal axis (124) and connected to the first beam (101) at the second end (101b) of said first beam,
a second portion (131b) flexible about an axis perpendicular to the principal axis (124) and connected to the second beam (102) at the first end (102a) of said second beam,
a first rigid portion (131d) connecting the first and second flexible portions (131a, 131b),
a second rigid portion (131e) adapted to be positioned against a second fixed point (112),
a third portion (131c) flexible about an axis perpendicular to the principal axis (124) connecting the second beam (102) to the second rigid portion (131e) at a pivot point (102c) of said second beam (102) so that the assembly formed by the second rigid portion (131e) and the second beam (102) forms a lever about said pivot point (102c),

said flexible portions (131a, 131b, 131c) and rigid portions (131d, 131e) being parts of a one-piece component.

**2.** The movement amplifying actuation device (100) as claimed in claim 1, further comprising:

- a third beam (103) comprising a piezoelectric element (P), adapted to flex about the principal axis (124) when a voltage is applied to it and having a first end (103a) and a second end (103b);
- a second articulation (132) comprising:

a first portion (132a) flexible about an axis perpendicular to the principal axis (124) and connected to the second beam (102) at the second end (102b) of said second beam,
a second portion (132b) flexible about an axis perpendicular to the principal axis (124) and connected to the third beam (103) at the first end (103a) of said third beam,
a first rigid portion (132d) connecting the first and second flexible portions (132a, 132b),
a second rigid portion (132e) adapted to be positioned against a third fixed point (113),
a third portion (132c) flexible about an axis perpendicular to the principal axis (124) connecting the third beam (103) to the second rigid portion (132e) at a pivot point (103c) of said third beam (103) so that the assembly formed by the second rigid portion (132e) and the second beam (132) forms a lever about said pivot point (103c),
said flexible portions (132a, 132b, 132c) and rigid portions (132d, 132e) being parts of a one-piece component.

3. The movement amplifying actuation device (100) as claimed in claim 1 or 2, comprising:

- a first beam (101) comprising a piezoelectric element (P), adapted to flex about a principal axis (124) when a voltage is applied to it and adapted to be attached at a first end (101a) to a first fixed point (111);
- N other beams (10M), N being greater than or equal to 2 and M varying between 2 and N, each $M^{th}$ beam comprising a piezoelectric element (P) and being adapted to flex about the principal axis (124) when a voltage is applied to it, and having a first end (10Ma) and a second end (10Mb); the $N^{th}$ beam (10N) having a free second end (10Nb);
- X articulations (13Y), X being equal to N-1 and Y being equal to M-1, each $Y^{th}$ articulation (13Y) comprising:

a first portion (13Ya) flexible about an axis perpendicular to the principal axis (124) and connected to the $Y^{th}$ beam (10Y) at the second end (10Yb) of said $Y^{th}$ beam,

a second portion (13Yb) flexible about an axis perpendicular to the principal axis (124) and connected to the $M^{th}$ (10M) at the first end (10Ma) of said $M^{th}$ beam,
a first rigid portion (13Yd) connecting the first and second flexible portions,
a second rigid portion (13Ye) adapted to be positioned against an $M^{th}$ fixed point (11M),
a third portion (13Yc) flexible about an axis perpendicular to the principal axis (124) connecting the $M^{th}$ beam (10M) to the second rigid portion (13Ye) at a pivot point (10Mc) of said $M^{th}$ beam (10M) so that the assembly formed by the second rigid portion (13Ye) and the $M^{th}$ beam (13M) forms a lever about said pivot point (10Mc);
said flexible portions (13Ya, 13Yb, 13Yc) and rigid portions (13Yd, 13Ye) being parts of a one-piece component.

4. The movement amplifying actuation device (100) as claimed in claim 3, in which at least one $Y^{th}$ articulation (13Y) further comprises a third rigid portion (13Yf) and a fourth rigid portion (13Yg) forming with the other parts of said articulation a one-piece component:

- the third rigid portion (13Yf) forming the connection between the first flexible portion (13Ya) of said $Y^{th}$ articulation (13Y) and the $Y^{th}$ beam (10Y),
- the fourth rigid portion (13Yg) forming the connection between the second flexible portion (13Yb) of said $Y^{th}$ articulation (13Y) and the $M^{th}$ beam (10M).

5. The movement amplifying actuation device (100) as claimed in any one of the preceding claims, in which the flexible portions of at least one $Y^{th}$ articulation (13Y) have parallel articulation axes perpendicular to the principal axis (124).

6. The movement amplifying actuation device (100) as claimed in any one of the preceding claims, in which the piezoelectric element (P) comprises lead zirconate titanate.

7. The movement amplifying actuation device (100) as claimed in any one of the preceding claims, in which at least one beam (10M) has a bimorph structure.

8. A spherical parallel kinematic microbot (60) with two degrees of freedom, comprising:

- an orientation device with two degrees of freedom comprising a platform (30) to be oriented about a first rotation axis (22) and a second rotation axis (23) relative to a fixed base (2), a first actuation arm (4) and a second actuation arm (8);

- first and second movement amplifying actuation devices (100, 100') as claimed in any one of claims 1 to 7;

the first actuation device (100) being connected to the first actuation arm (4) so as to transmit to it a first movement in translation relative to the fixed base (2) so as to drive the platform (30) in rotation about the first axis (22), and

the second actuation device (100') being connected to the second actuation arm (8) so as to transmit to it a second movement in translation relative to the fixed base (2) so as to drive the platform (30) in rotation about the second axis (23).

9. The microbot (60) as claimed in claim 8, in which the orientation device is a spherical orientation device (1) with two degrees of freedom connecting the platform (30) to two fixing points (2a, 2b) of a fixed base (2) so as to be able to orient said platform (30) in space by rotation about a first axis (22) and a second axis (23), these two axes (22, 23) being substantially perpendicular and crossing at a center of spherical movement (21) situated in said member (30) to be oriented, comprising:

- a first actuation arm (4) configured to effect a movement in translation relative to the fixed base (2) and adapted to apply to a first transmission arm (5) connected to a first fixing point (2a) of the fixed space (2) by a flexible connection (6) articulated about the first axis (22) a movement in rotation relative to said fixed base (2) so as to transmit to the platform (30) a movement in rotation about said first axis (22);
- an intermediate arm (12) connected to the first transmission arm (5) by a flexible connection (13) articulated about a third axis (24) perpendicular to the first and second axes (22) and (23) and connected to the platform (30) by a flexible connection (14) so as to transmit to the platform (30) a movement in rotation about the first axis (22);
- a second actuation arm (8) configured to effect a movement in translation relative to the fixed base (2) and adapted to apply to a second transmission arm (9) connected to a second fixing point (2b) of the fixed base (2) by a flexible connection (10) articulated about the second axis (23) a movement in rotation relative to said fixed base (2) so as to apply to the platform (30) a movement in rotation about said second axis (23), said arm (9) being connected to the platform (30) by a flexible connection (15, 16) articulated about the first axis (22) so as not to drive said second transmission arm (9) in rotation about the first axis (22) during actuation of the first actuation arm (4);

the connection (14) between the platform (30) and intermediate arm (12) being articulated about the axis (23) so as not to drive said intermediate arm (12) in rotation about the second axis (23) during actuation of the second actuation arm (8); and the arms (4, 5, 8, 9, 12), the flexible connections (6, 10, 13, 14, 15, 16) and the platform (30) forming the parts of a one-piece component forming the device (1), the arms (4, 5, 8, 9, 12) and the platform (30) being rigid portions of the device (1), and the flexible connections (6, 10, 13, 14, 15, 16) being flexible portions each forming a hinge about one only of the first, second and third axes (22, 23, 24), and connecting said rigid portions to one another, to the fixed base (2).

10. The microbot (60) as claimed in claim 8 or 9, in which the rigid portions (4, 5, 8, 9, 12, 30) comprise a central layer (41) of a flexible material, such as a polyimide, sandwiched between two layers (42, 43) of a rigid material, such as carbon fiber, the flexible connections (6, 10, 13, 14, 15, 16) being composed of the central layer.

11. The microbot (60) as claimed in any one of claims 8 to 10, in which one or more transmission arms (5, 9) and/or intermediate arms (12) form a circular arc.

12. The microbot (60) as claimed in any one of claims 8 to 11, in which the spherical orientation device (1) further comprises a flexible connection (7) articulated about an axis parallel to the first axis (22) and disposed between the first actuation arm (4) and the first transmission arm (5) and forming with the arms (4, 5, 8, 9, 12), the flexible connections (6, 10, 13, 14, 15, 16) and the platform (30) a one-piece component.

13. The microbot (60) as claimed in any one of claims 8 to 12, the first actuation arm (4) comprising a first portion (4a) adapted to be coupled to the actuation device (100) and a second portion (4b) connected to the first transmission arm (5) by a flexible connection (7) articulated about an axis parallel to the first axis (22), said first and second portions (4a) and (4b) being connected by a flexible connection (17) articulated about an axis parallel to the first axis (22) and forming with the arms (4, 5, 8, 9, 12), the flexible connections (6, 7, 10, 13, 14, 15, 16) and the platform (30) a one-piece component.

14. The microbot (60) as claimed in any one of claims 8 to 13, the spherical orientation device (1) further comprising a flexible connection (11) connecting the second actuation arm (8) and the second transmission arm (9) articulated about an axis parallel to the second axis (23) and forming with the arms (4, 5, 8, 9, 12), the flexible connections (6, 10, 13, 14, 15, 16) and the platform (30) a one-piece component.

15. The microbot (60) as claimed in any one of claims 8 to 14, in which the spherical orientation device (1) as claimed in any one of claims 1 to 5 further comprises a universal joint type double flexible connection (19) connecting the second actuation arm (8) and the second transmission arm (9) articulated about an axis parallel to the first axis (22) and an axis parallel to the second axis (23) and forming with the arms (4, 5, 8, 9, 12), the flexible connections (6, 10, 13, 14, 15, 16) and the platform (30) a one-piece component.

16. The microbot (60) as claimed in any one of claims 8 to 15, in which the second actuation arm (8) comprises a first portion (8a) adapted to be coupled to the actuation device (100') and a second portion (8b) connected to the second transmission arm (9) by a single flexible connection (11) or a double flexible connection (19), the first and second portions (8a) and (8b) being connected to a universal joint type double flexible connection (18) articulated about an axis parallel to the first axis (22) and an axis parallel to the second axis (23), and forming with the arms (4, 5, 8, 9, 12), the flexible connections (6, 10, 13, 14, 15, 16) and the platform (30) a one-piece component.

17. The microbot (60) as claimed in any one of claims 8 to 16, further comprising a support (50) configured to create the fixed point of the actuation devices (100, 100').

18. The microbot (60) as claimed in any one of claims 8 to 17, in which the platform (30) supports a mirror (31).

19. The microbot (60) as claimed in claim 18, in which the mirror (31) is disposed so as to reflect a laser beam (71).

20. The microbot (60) as claimed in claim 19, further comprising a device for viewing a spot of the laser beam (71) positioned on a surface.

FIG.1

FIG.2a

FIG.2b

FIG.2c

FIG.3a

FIG.3b

FIG.4a

FIG.4b

FIG.5

FIG.6a

FIG.6b

41, 42

46, 47

## FIG.7a

41a, 42a

46, 47

## FIG.7b

41a          43    41

46

## FIG.7c

47    42

42a          43

41

46

41a

## FIG.7d

42

43

41

## FIG.7e

FIG.8a

FIG.8b

FIG.8c

FIG.9a

FIG.9b

FIG.10

FIG.12

FIG.11a

FIG.11b

FIG.11c

FIG.11d

FIG.13

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2850218
- FR 2362525